# EUROPEAN PATENT APPLICATION

(11) **EP 4 729 609 A2**
(43) Date of publication of application: **22.04.2026**
(21) Application number: 26160729.5
(22) Date of filing: 09.11.2021
(51) Int. Cl.: C12N 5/0783

(54) **METHOD FOR CRYOPRESERVING ENGINEERED TREGS**

(30) Priority: 09.11.2020 GB 202017678; 25.05.2021 GB 202107418
(62) Divisional of application: 21806262.8
(71) Applicant: Quell Therapeutics Limited, London W12 0BZ (GB)
(72) Inventor: MCGILL, Iain, London W12 0BZ (GB)
(74) Representative: Dehns

(57) **Abstract**

The present invention relates to a method of preserving CD62L expression in a regulatory T cell (Treg) population that has been cryopreserved, comprising introducing a polynucleotide encoding a FOXP3 polypeptide into the Treg population prior to cryopreservation. The present invention also relates to a method of preserving CD62L in a Treg population after cryopreservation, comprising introducing a polynucleotide encoding a FOXP3 polypeptide into the Treg population and cryopreserving said Treg population. Furthermore, the present invention relates to the use of an exogenous polynucleotide encoding FOXP3 for the preservation of CD62L expression in a Treg population after cryopreservation, and to a cryopreserved engineered Treg, pharmaceutical compositions comprising the cryopreserved engineered Treg and to therapeutic uses thereof.

## Description

### FIELD OF THE INVENTION

The present invention relates to a method of cryopreserving regulatory T cells (Tregs). In particular, the present invention relates to a method of increasing FOXP3 expression in Tregs prior to cryopreservation, to preserve the suppressive function of the Tregs post cryopreservation. The present invention also relates to a cryopreserved engineered Treg, pharmaceutical compositions comprising the cryopreserved engineered Treg and to therapeutic uses thereof.

### BACKGROUND TO THE INVENTION

In recent years, there has been increasing interest in using regulatory T cells (Tregs) in the clinical setting for adoptive cell therapy (ACT) to treat a range of different conditions. Tregs have been proposed for use in controlling undesired immune responses based on their immunosuppressive function. For example, Tregs have been used for the treatment of autoimmune or allergic diseases, for immunomodulation in transplantation, and to improve and/or prevent immune-mediated organ damage in inflammatory disorders. In addition, Tregs have been genetically engineered to express T cell receptors (TCRs) or chimeric antigen receptors (CARs) with new specificities, offering the advantage of providing targeted immune suppression. Various phase I trials have shown that Treg therapy is safe and efficacious and there are currently several phase II trials underway.

The main source of Tregs for therapeutic use is from a patient's own blood (drawn directly from a blood vessel or as a product of leukapheresis) or from umbilical cord blood. The number of Tregs from these sources is low and substantial numbers are required to effectively suppress the immune system. Therefore, *ex-vivo* expansion is necessary to obtain adequate numbers of Tregs for infusion into the patient. A typical isolation and expansion protocol that follows Good Manufacturing Practice (GMP) guidelines takes from approximately nine days up to twenty-one days. It would be highly advantageous if these cells could be processed and frozen in sufficient quantity, ready to be used in the patient when needed, avoiding prolonged processing which can have a detrimental effect on Treg quality. This approach would allow for more flexibility in terms of planning the therapy, timing of the infusions, would allow for subsequent Treg infusions and collection of cells prior to pharmacological therapy. Therefore, the ability to cryopreserve Tregs is of vital importance.

The effects of freezing and thawing on the Treg cell population are not well defined. Several research groups have reported that cryopreservation can have a detrimental influence on Tregs and can decrease Treg viability, cause abnormal cytokine secretion, and alter the expression of surface markers essential for proper Treg suppressive function and migration.

**In** particular, Florek et al. (Florek et al, PLoS One, 2015, DOI: 10.137/journal.pone.0145763) have shown that freeze-thaw of Tregs results in loss of CD62L (L-selectin) expression and an impaired ability to protect against graft-versus-host-disease (GvHD). The thawed Tregs showed reduced capacity to bind to the CD62L binding partner MADCAM1 and impaired homing to secondary lymphoid organs. Other studies have shown that CD62L (L-selectin) and CCR5 expression on T cells is lost after cryopreservation (De Boer et al, Bone Marrow Transplant, 1998, 22:1103-10 and Hattori et al, Exp Hematol, 2001, 29:114-22). Tregs lacking expression of CD62L have reduced trafficking abilities, localisation and consequently the compartmentalisation of Treg-controlled immune responses (Sakaguchi et al, Cell, 2008, 133:775-87). Loss of CD62L expression after thawing can be reversed by overnight culture (De Boer et al., 1998 and Hattori et al., 2001). However, resting the cells in this way may not be practical for clinical applications, where thawing is usually carried out at the bedside and thus further culture is not possible.

Golab et al. (Golab et al, Oncotarget, 2018, vol.9,(No.11),pp:9728-9740) evaluated two cell-banking strategies for Treg therapy; cryopreservation of CD4+ cells for subsequent Treg isolation/expansion and cryopreservation of *ex-vivo* expanded Tregs (CD4⁺CD25^{hi}CD127^{lo/-} cells). *Ex-vivo* expanded Tregs were found to be more sensitive to the cryopreservation process than CD4+ cells, with much lower cell viability after thawing and a decrease in Treg marker expression (i.e. a decrease in the frequency of cells with the phenotype CD4⁺CD25^{hi}CD127⁻ and CD4⁺FoxP3⁺). Poor Treg viability and impaired phenotype after thawing was overcome by re-stimulating Tregs during subsequent *ex-vivo* expansion for 13 days. Similarly, Peters et al. (Peters et al, PLoS One, 2008, 3:e3161) showed that Treg suppressive capacity after thawing can be recovered by stimulation and expansion for 10 days.

However, as discussed above, in a clinical setting these additional culture and expansion steps are not practicable as well as taking time and coming with added risks, negating the benefit of using cryopreserved Tregs versus freshly isolated cells. Consequently, there remains a need for improved methods of cryopreservation, where cryopreserved Tregs can be administered immediately to the patient after thawing, without the lengthy post-thawing rescue steps that are currently required in the laboratory.

### SUMMARY OF THE INVENTION

In this regard, the present inventors have surprisingly found that exogenous FOXP3 expression provides a protective effect on Treg phenotype after cryopreservation. The inventors have determined that Tregs transduced with FOXP3 maintain CD62L (or L-selectin) expression after freezing and thawing. CD62L expression is essential for the migratory and homing functions of Tregs and is therefore important for their immunosuppressive function. Thus, transducing Tregs with FOXP3 prior to cryopreservation (e.g. as part of the necessary processing of the cells) results in Tregs that maintain their immunoregulatory and suppressive functions post-thawing, allowing said cells to be used immediately in the patient. The inventors have thus provided a solution to the problem associated with the cryopreservation of Tregs in a manner that avoids further culture of the cells and a path for producing Treg therapies that can be effectively used in the clinic.

Accordingly, the invention provides engineered Tregs that maintain their immunosuppressive properties after cryopreservation and thus have enhanced clinical efficacy and safety. The cryopreserved Tregs may be used for a variety of therapeutic uses, including inducing tolerance to a transplant in a subject or for the treatment and/or prevention of transplant rejection, graft-vs-host-disease, inflammation, an autoimmune disease, an allergic disease, a neuroinflammatory disease such as amyotrophic lateral sclerosis (ALS), a metabolic disease such as type I diabetes mellitus or for suppressing an immune response.

In a first aspect, the invention provides a method of preserving CD62L expression in a regulatory T cell (Treg) population or Treg that has been cryopreserved, comprising introducing a polynucleotide encoding a FOXP3 polypeptide into the Treg population or Treg prior to cryopreservation.

The Treg population or Treg may have higher CD62L expression after cryopreservation than a corresponding non-engineered Treg population or Treg (i.e. a Treg population or Treg that has not been transduced with a polynucleotide encoding FOXP3) after cryopreservation.

The method may additionally comprise the step of cryopreserving said Treg population or Treg.Thus, the invention may further provide a method of preserving CD62L expression in a regulatory T cell (Treg) population or Treg for cryopreservation comprising the steps of (a) introducing a polynucleotide encoding a FOXP3 polypeptide into the Treg population or Treg and (b) cryopreserving said Treg population or Treg.

Alternatively, the invention may provide a method for cryopreservation of a Treg (or a population of Tregs) comprising (a) introducing a polynucleotide encoding FOXP3 into a Treg or Treg population and (b) cryopreserving said Treg or Treg population, wherein said cryopreserved Treg or Treg population has a higher expression level of CD62L as compared to a corresponding non-engineered Treg or Treg population.

**In** another aspect, the invention provides a method for producing a cryopreserved Treg or population of Tregs having a level of CD62L comparable to a corresponding non-cryopreserved Treg or population of Tregs comprising (a) introducing a polynucleotide encoding FOXP3 into a Treg or population of Tregs and (b) cryopreserving said Treg or population of Tregs.

A skilled person will appreciate that method steps described herein are typically provided in their order of action, e.g. step (a) should be performed before step (b).

The methods of the invention may further include any one or more of the additional steps of isolating a Treg or Treg population from a sample prior to introducing the polynucleotide encoding a FOXP3 into said Treg or Treg population, thawing said Treg or Treg population and/or administering the thawed Treg or Treg population to a subject. The methods may further include a step of expanding the Treg population or Treg prior to cryopreservation. Alternatively viewed, the Treg or Treg population of the invention may not be cryopreserved until it has been expanded. The cryopreserved Treg or Treg population of the present invention may be used immediately, as an off-the-shelf therapeutic product, without the need for further expansion, modification, or alternative additional culture steps.

**In** this respect, the invention additionally provides a method for preserving CD62L expression in a Treg or Treg population that has been thawed from a cryopreserved state comprising introducing a polynucleotide encoding a FOXP3 polypeptide into the Treg or Treg population prior to cryopreservation and thawing.

Alternatively viewed, the invention provides a method of preserving CD62L expression in a Treg or Treg population after thawing from a cryopreserved state comprising (a) introducing a polynucleotide encoding FOXP3 into a Treg or Treg population, (b) cryopreserving said Treg or Treg population and (c) thawing said Treg or Treg population. The Treg or Treg population may be used immediately after/during thawing without additional expansion. The method may further comprise the steps of (i) enriching Tregs from a sample suspected of containing Tregs, to produce a Treg-enriched sample, prior to step (a), and (ii) expanding the Treg or Treg population from the Treg-enriched sample after step (a) but before step (b) to produce an engineered expanded Treg or Treg population. Step (i) may further comprise depleting CD8+ cells.

In another aspect, the invention provides a method for producing a thawed Treg or population of Tregs having a level of CD62L comparable to a corresponding non-cryopreserved Treg or population of Tregs comprising (a) introducing a polynucleotide encoding FOXP3 into a Treg or Treg population, (b) cryopreserving said Treg or Treg population and (c) thawing said Treg or Treg population. The Treg or Treg population may be used immediately after/during thawing without additional expansion. The method may further comprise the steps of (i) enriching Tregs from a starting cell sample suspected of containing Tregs, to produce a Treg-enriched sample, prior to step (a), and (ii) expanding the Treg or Treg population from the Treg-enriched sample after step (a) but before step (b) to produce an engineered expanded Treg or Treg population. Step (i) may further comprise depleting CD8+ cells.

The sample (also known as a Treg cell-containing sample or simply a cell-containing sample) may comprise or consist of whole blood, umbilical cord blood, leukocyte cones, blood cones, peripheral blood mononuclear cells (PBMCs) or leukopaks. Typically, the sample may comprise or consist of leukocyte cones or one or more leukopaks. Typically, the sample is from a human donor. The donor may be healthy or may have a disease, e.g., a neurogenerative disease (such as ALS), or an autoimmune disease (such as type I diabetes) or may be a transplant patient.

The cryopreservation of said Treg population (or Treg) may comprise the following steps of: (bi) suspending said Treg population in a cryopreservation media; (bii) freezing the Treg population from (bi); and (biii) storing said Treg population from (bii) at a temperature below - 130°C. Suitable cryopreservation media may be any cryopreservation media known in the art, as discussed in detail below. Preferably, the cryopreservation media will contain one or more cryoprotectants and suitable cryoprotectants are discussed below. Preferably, the cryopreservation media and cryoprotectant(s) will comply with Good Manufacturing Practice (GMP) standards.

The methods of the invention may further comprise any one or more of the steps of: pre-chilling said Treg population (or Treg), and/or any one or more (and preferably all) reagents or devices to be used in any step of cryopreservation, prior to cryopreservation (step (b)); freezing said Treg population (or Treg) (according to step (b)) at a controlled rate of freezing of approximately -1°C per minute; and/or storing the Treg population (or Treg) at -80°C for up to 24 hours prior to step (biii). "Reagents" may include, for example, the cryopreservation media, and "devices" may include, for example, any freezing device that maintains a controlled rate of freezing. Suitable reagents and devices may be any reagents or devices used for cryopreservation that are known in the art. Preferably, such reagents and devices will comply with GMP standards.

Step (biii) may comprise storing said Treg population in liquid nitrogen. The temperature of liquid nitrogen may be approximately -196°C.

The step of thawing said Treg population (or Treg) may comprise warming the Treg composition from a temperature below -130°C to a temperature between approximately 0 to 10°C, optionally wherein warming the Treg population comprises placing the Treg composition in a water bath maintained at approximately 37°C.

The cryopreservation and/or thawing steps may be carried out in a closed system or Grade A environment, in accordance with GMP standards.

The Treg population may be isolated from a sample by selecting for CD4⁺CD25⁺CD127⁻ and/or CD4⁺CD25⁺CD127^{low} cells or by selecting for CD4⁺CD25^{hi}CD127⁻ and/or CD4⁺CD25⁺CD127^{low} cells. The Treg population may be isolated by selecting for CD45RA⁺ cells, preferably CD4⁺CD25⁺CD127^{low}CD45RA⁺ cells. As such, the cryopreserved Treg population of the invention may comprise at least 70%, at least 80%, at least 90%, or at least 95% CD4⁺CD25⁺CD127⁻, CD4⁺CD25⁺CD127^{low} and/or CD4⁺CD25⁺CD127^{low}CD45RA⁺ Treg cells.

Furthermore, the cryopreserved Treg population may comprise fewer than 20% CD8+ cells, fewer than 10% CD8+ cells, preferably fewer than 5% CD8+ cells and more preferably fewer than 2% CD8+ cells. **In** this regard, the methods of the present invention may comprise a step of depleting CD8+ cells prior to the transduction step (i.e., prior to step (a) of the above methods, which comprises introducing a polynucleotide encoding FOXP3 into a Treg or Treg population).

**In** a second aspect, the invention provides use of an exogenous polynucleotide encoding FOXP3 for the preservation of CD62L expression in a Treg population or Treg that has been cryopreserved or is for cryopreservation. Particularly, as discussed above, the exogenous polynucleotide is introduced into the Treg population or Treg, and its expression provides a protective effect on CD62L expression levels within the Treg population or Treg.

A third aspect of the invention provides a cryopreserved engineered Treg population or Treg comprising an exogenous polynucleotide encoding a FOXP3 polypeptide, wherein the engineered Treg population or Treg has higher CD62L expression level after cryopreservation than a corresponding non-engineered Treg population or Treg after cryopreservation.

The invention further provides a Treg population or Treg obtainable or obtained by a method of the invention (e.g. a cryopreserved or thawed cell). Alternatively viewed, the invention provides a thawed Treg or Treg population comprising an exogenous polynucleotide encoding a FOXP3 polypeptide, wherein the engineered Treg or Treg population has higher CD62L expression level after thawing than a corresponding non-engineered Treg or Treg population after thawing.

The FOXP3 polypeptide may comprise an amino acid sequence which is at least 80% identical to SEQ ID NO. 1 or a functional fragment thereof. The polynucleotide encoding FOXP3 may be within an expression vector.

The methods may further comprise introducing a polynucleotide encoding an exogenous T cell receptor (TCR) or a polynucleotide encoding a chimeric antigen receptor (CAR) into the Treg population or Treg, or the cryopreserved engineered Treg population or Treg may comprise a polynucleotide encoding an exogenous T cell receptor (TCR) or a polynucleotide encoding a chimeric antigen receptor (CAR). The polynucleotide encoding the FOXP3 polypeptide and the polynucleotide encoding the exogenous TCR or CAR may be provided by a single expression vector.

The TCR or CAR may be directed to any desired target molecule, particularly to a target molecule expressed on a target cell. Suitable TCRs and CARs are discussed below. In an embodiment, the CAR is directed against an HLA antigen, for example HLA-A2.

The vector may comprise a first polynucleotide encoding the FOXP3 polypeptide and a second polynucleotide encoding the exogenous TCR or CAR, wherein the first polynucleotide and the second polynucleotide are operably linked to the same promoter, and wherein the first polynucleotide is upstream of the second polynucleotide. There may be an internal self-cleaving sequence between the polynucleotide encoding FOXP3 and the polynucleotide encoding the exogenous TCR or CAR. Suitable promoters and self-cleaving sequences are discussed below.

The methods of the invention may further comprise introducing a polynucleotide encoding a safety switch comprising a suicide moiety into the Treg population or Treg or the cryopreserved engineered Treg population or Treg of the invention may comprise a safety switch comprising a suicide moiety. Thus, in one embodiment, the polynucleotides/nucleic acid molecules encoding FOXP3 and optionally a CAR/TCR may also encode a safety switch comprising a suicide moiety. Particularly, the nucleic acid molecules and constructs may be designed to encode the separate components (e.g. three components (e.g. FOXP3, exogenous TCR or CAR and safety switch)) within a single nucleic acid molecule, or construct, such that and the components may be produced in the cell as individual components, i.e. as discrete entities (i.e. are not linked to each other and are physically distinct). Thus, the expressed components encoded by the nucleic acid molecule may be separately located, in or on the cell, as separate and distinct, or discrete, functional polypeptides. This is achieved by encoding cleavage sequences in the nucleic acid molecule, in particular self-cleaving sequences, in between the nucleotide sequences encoding the respective components.

The polynucleotides disclosed herein (e.g. encoding FOXP3 and/or the exogenous TCR or CAR and/or the safety switch) may be introduced into the Treg population or Treg by viral transduction, preferably retroviral or lentiviral transduction.

**In** a fourth aspect, the invention provides a pharmaceutical composition comprising a cryopreserved engineered Treg population or Treg, or thawed Treg or Treg population as described herein.

**In** a fifth aspect, the invention provides a cryopreserved engineered Treg population or Treg as described herein, thawed Treg or Treg population or a pharmaceutical composition as described herein for use in prevention and/or treatment of a disease.

**In** a sixth aspect, the invention provides use of a cryopreserved engineered Treg population or Treg, thawed Treg or Treg population or a pharmaceutical composition as described herein in the manufacture of a medicament for prevention and/or treatment of a disease.

**In** a seventh aspect, the invention provides a method of prevention and/or treatment of a disease comprising administering to a subject a cryopreserved engineered Treg population or Treg as described herein, thawed Treg or Treg population or a pharmaceutical composition as described herein.

The disease may be an autoimmune disease, allergic disease, transplant rejection, graft-vs-host-disease, inflammation, a neuroinflammatory disease, such as amyotrophic lateral sclerosis (ALS) or a metabolic disease such as diabetes mellitus (e.g. type I diabetes mellitus). The present invention also relates to a cryopreserved engineered Treg population or Treg as described herein, or a pharmaceutical composition as described herein for use, or use of a cryopreserved engineered Treg population or Treg or pharmaceutical composition as described herein, in suppressing an immune response.

**In** an embodiment, there is provided a method of improving the homing ability of a Treg or Treg population to secondary lymphoid organs after cryopreservation as compared to a corresponding non-engineered Treg or Treg population after cryopreservation, comprising the step of introducing a polynucleotide encoding a FOXP3 polypeptide into the Treg or Treg population prior to cryopreservation.

**In** a further embodiment, there is provided a method for deleting a cryopreserved Treg or population of Tregs as defined herein comprising a nucleic acid molecule/polynucleotide, expression construct, or vector encoding FOXP3, and a safety switch, which comprises the step of exposing the cryopreserved Treg or Treg population to a separate cell-deleting agent (such as an antibody) that recognises the suicide moiety within the safety switch. By binding to the suicide moiety, the cell-deleting agent may be targeted to the cell to be deleted. The method may be an *in vitro* method.

The invention may also provide a method for increasing the stability and/or suppressive function of a Treg or Treg population after cryopreservation comprising the step of introducing a nucleic acid molecule/polynucleotide, an expression construct or vector as provided herein into the cell prior to cryopreservation.

The methods of the present invention provide *ex-vivo* expanded cryopreserved therapeutic Tregs or Treg populations which, following thawing and without further expansion, maintain the high viability, purity, and potency seen in expanded, engineered Tregs or Treg populations prior to cryopreservation. Thus, the cells of the present invention may be used as off-the-shelf therapeutics.

The protection sought for the invention is as defined in the claims.

### DESCRIPTION OF DRAWINGS

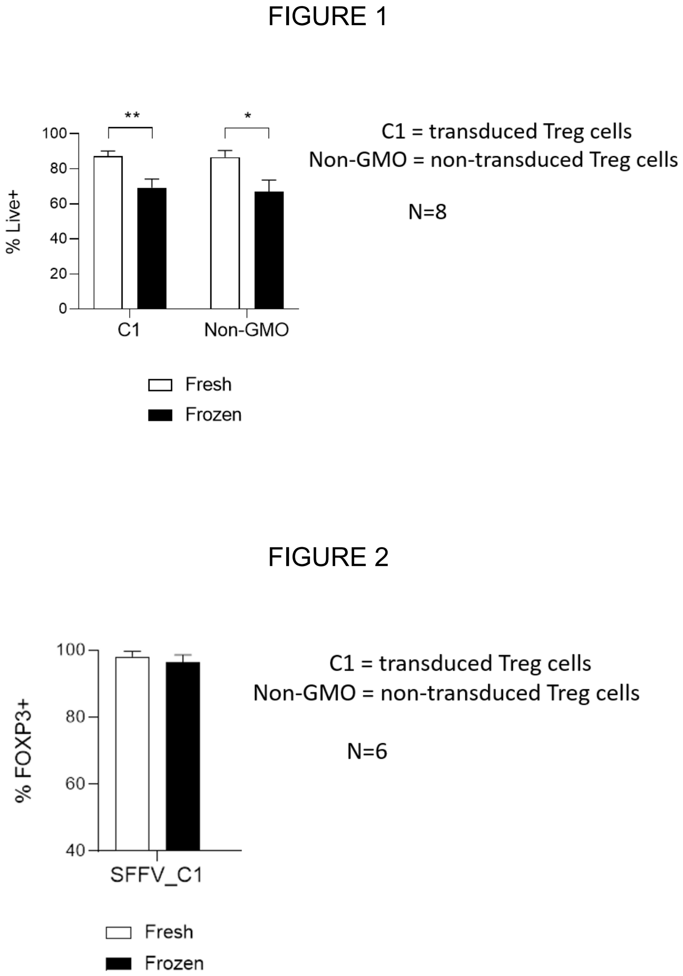

**Figure 1** **- Viability**
   Figure 1 shows the viability of fresh Tregs (i.e. non-frozen Tregs) and frozen Tregs that have been transduced with a construct expressing FOXP3 (C1) compared to the viability of fresh Tregs and frozen Tregs that that have not been transduced, i.e. not genetically modified (Non-GMO). In both transduced and non-transduced Tregs, around 75% of cells are still viable after freezing.
**Figure 2** - **Transduction**
   Figure 2 shows that both fresh and frozen Tregs transduced with C1 express FOXP3.

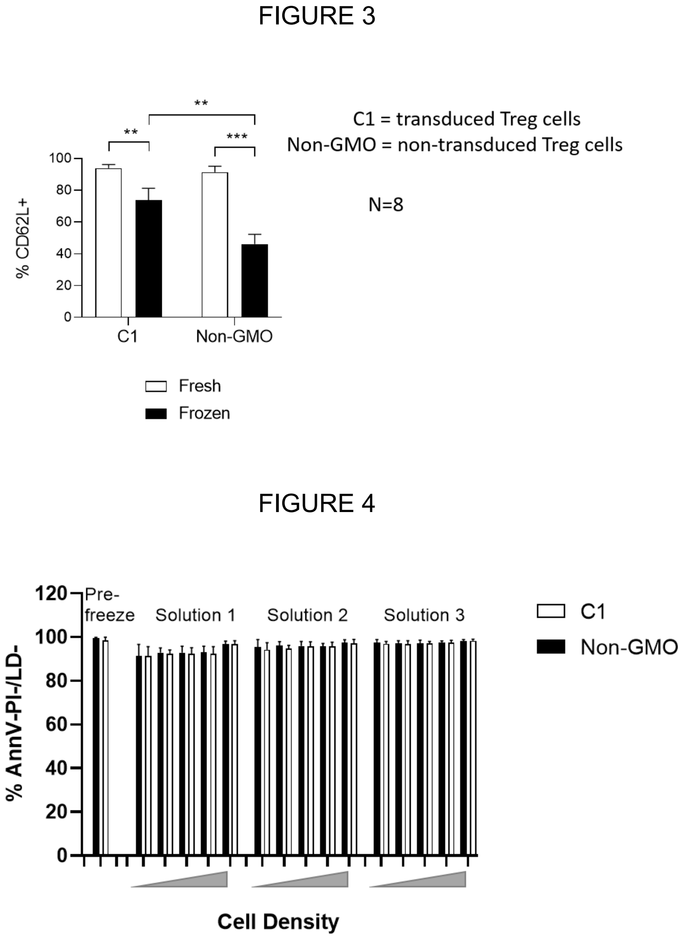

**Figure 3** - **Cell surface expression of CD62L**
   Figure 3 shows the cell surface expression of CD62L in fresh Tregs (i.e. non-frozen Tregs) and frozen Tregs that have been transduced with a construct expressing FOXP3 (C1) and the cell surface expression of CD62L in fresh and frozen Tregs that have not been transduced (Non-GMO). The percentage expression of CD62L is significantly increased in frozen transduced Tregs compared to frozen non-transduced Tregs.
**Figure 4** **- Viability in different cryopreservation solutions**
   Figure 4 shows the viability of fresh Tregs (i.e. non-frozen Tregs) ('pre-freeze' columns) and frozen Tregs, both of which were either transduced with a construct expressing FOXP3 (C1) or non-transduced (Non-GMO) (the non-transduced Tregs do not express exogenous FOXP3). The frozen Tregs were frozen in three different cryopreservation solutions: Solution 1, Solution 2 and Solution 3, at five different cell densities per solution. Viability was close to 100% in all conditions.

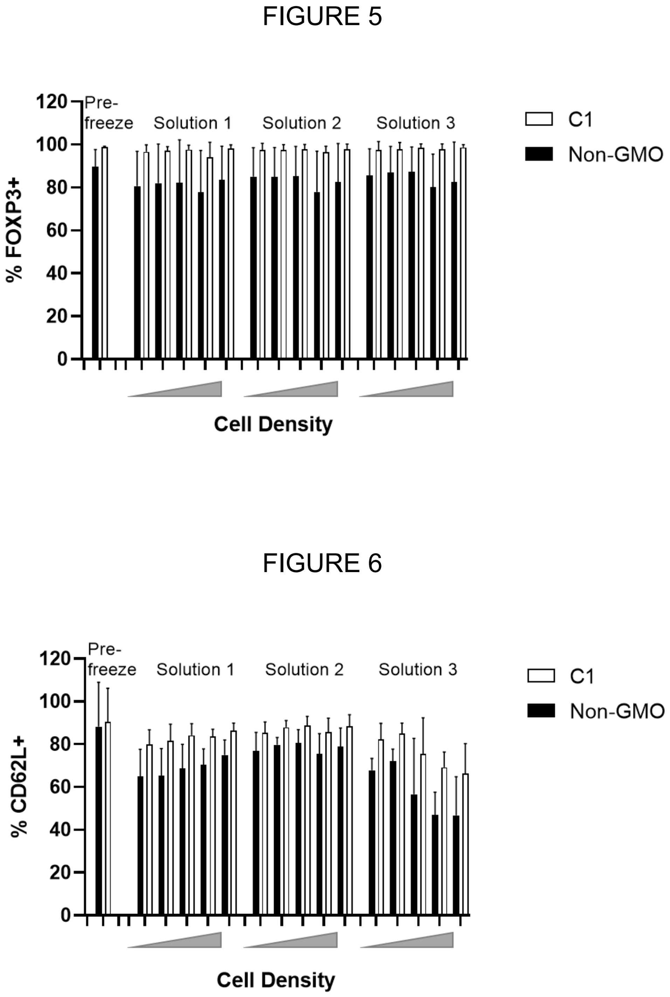

**Figure 5** - **Transduction in different cryopreservation solutions**
   Figure 5 shows transduction in fresh Tregs that have either been transduced with a construct expressing FOXP3 (C1) or non-transduced (Non-GMO) ('pre-freeze' columns), and in Tregs that have been transduced with C1 or non-transduced (Non-GMO) and then frozen in cryopreservation solution 1, 2 or 3 at five different cell densities per solution. In both the fresh and frozen conditions, Tregs transduced with C1 expressed high levels FOXP3. This high expression level was maintained in the frozen Tregs across all three different cryopreservation solutions 1, 2 and 3 and across all five different cell densities.
**Figure 6** - **Cell surface expression of CD62L in different cryopreservation solutions**
   Figure 6 shows the cell surface expression of CD62L in fresh Tregs that have either been transduced with a construct expressing FOXP3 (C1) or non-transduced (Non-GMO) ('pre-freeze' columns), and in Tregs that have been transduced with C1 or non-transduced (Non-GMO) and then frozen in cryopreservation solution 1, 2 or 3 at five different cell densities per solution. The percentage expression of CD62L is increased in Tregs transduced with C1 compared to non-transduced Tregs and is comparable across all three cryopreservation solutions and all five different cell densities.

### DETAILED DESCRIPTION

The present inventors have surprisingly found that transduction of regulatory cells with exogenous FOXP3 preserves their expression of CD62L at a level that allows maintenance of regulatory function after cryopreservation. Accordingly, the present invention provides a method of preserving CD62L expression in a regulatory T cell (Treg) population (or Treg) that has been cryopreserved, comprising introducing a polynucleotide encoding a FOXP3 polypeptide into the Treg population prior to cryopreservation. Alternatively viewed, the present invention provides a method for producing a cryopreserved Treg or population of Tregs having a level of CD62L comparable to a corresponding non-cryopreserved Treg or population of Tregs comprising (a) introducing a polynucleotide encoding FOXP3 into a Treg or population of Tregs and (b) cryopreserving said Treg or population of Tregs. The present invention also provides a cryopreserved engineered Treg population (or Treg) comprising an exogenous polynucleotide encoding a FOXP3 polypeptide, wherein the engineered Treg population has higher CD62L expression after cryopreservation than a corresponding non-engineered Treg population after cryopreservation (or alternatively, a comparable level of CD62L expression to a corresponding non-cryopreserved Treg or Treg population).

### REGULATORY T CELLS

"Regulatory T cells (Treg) or T regulatory cells" are immune cells with immunosuppressive function that control cytopathic immune responses and are essential for the maintenance of immunological tolerance. As used herein, the term Treg refers to a T cell with immunosuppressive function.

A T cell as used herein is a lymphocyte including any type of T cell, such as an alpha beta T cell (e.g. CD8 or CD4+), a gamma delta T cell, a memory T cell or a Treg cell.

Suitably, immunosuppressive function and the term "immune response" may refer to the ability of the Treg to reduce or inhibit one or more of a number of physiological and cellular effects facilitated by the immune system in response to a stimulus such as a pathogen, an alloantigen, or an autoantigen. Examples of such effects include increased proliferation of conventional T cell (Tconv) and secretion of proinflammatory cytokines. Any such effects may be used as indicators of the strength of an immune response. A relatively weaker immune response by Tconv in the presence of Tregs would indicate an ability of the Treg to suppress immune responses. For example, a relative decrease in cytokine secretion would be indicative of a weaker immune response, and thus indicative of the ability of Tregs to suppress immune responses. Tregs can also suppress immune responses by modulating the expression of co-stimulatory molecules on antigen presenting cells (APCs), such as B cells, dendritic cells and macrophages. Expression levels of CD80 and CD86 can be used to assess suppression potency of activated Tregs *in vitro* after co-culture.

Assays are known in the art for measuring indicators of immune response strength, and thereby the suppressive ability of Tregs. In particular, antigen-specific Tconv cells may be co-cultured with Tregs, and a peptide of the corresponding antigen added to the co-culture to stimulate a response from the Tconv cells. The degree of proliferation of the Tconv cells and/or the quantity of the cytokine IL-2 they secrete in response to addition of the peptide may be used as indicators of the suppressive abilities of the co-cultured Tregs.

Antigen-specific Tconv cells co-cultured with Tregs (or a Treg population) as disclosed herein may proliferate 5%, 10%, 20%, 30%, 40%, 50%, 60%, 70%, 90%, 95% or 99% less than the same Tconv cells cultured in the absence of the Tregs. For example, antigen-specific Tconv cells co-cultured with the present cryopreserved Tregs may proliferate 5%, 10%, 20%, 30%, 40%, 50%, 60%, 70%, 80%, 90%, 95% or 99% less than the same Tconv cells cultured in the presence of non-engineered cryopreserved Tregs.

Antigen-specific Tconv cells co-cultured with the Tregs herein may express at least 10%, at least 20%, at least 30%, at least 40%, at least 50%, or at least 60% less effector cytokine than corresponding Tconv cells cultured in the absence of the Tregs (e.g. in the presence of non-engineered cryopreserved Tregs). The effector cytokine may be selected from IL-2, IL-17, TNFα, GM-CSF, IFN-γ, IL-4, IL-5, IL-9, IL-10 and IL-13. Suitably the effector cytokine may be selected from IL-2, IL-17, TNFα, GM-CSF and IFN-γ.

Several different subpopulations of Tregs have been identified which may express different or different levels of particular markers. Tregs generally are T cells which express the markers CD4, CD25 and FOXP3 (CD4⁺CD25⁺FOXP3⁺). Tregs may also express CTLA-4 (cytotoxic T-lymphocyte associated molecule-4) or GITR (glucocorticoid-induced TNF receptor).

Treg cells are present in the peripheral blood, lymph nodes, and tissues and Tregs for use herein include thymus-derived, natural Treg (nTreg) cells, peripherally generated Tregs, and induced Treg (iTreg) cells.

A Treg may be identified using the cell surface markers CD4 and CD25 in the absence of, or in combination with, low-level expression of the surface protein CD127 (CD4⁺CD25^{+/hi}CD127⁻ or CD4⁺CD25⁺CD127^{low}). The use of such markers to identify Tregs is known in the art and described in Liu et al. (JEM; 2006; 203; 7(10); 1701-1711), for example.

A Treg may be a CD4⁺CD25⁺FOXP3⁺ T cell, a CD4⁺CD25⁺CD127⁻ T cell, or a CD4⁺CD25⁺FOXP3⁺CD127^{-/low} T cell.

Suitably, the Treg may be a natural Treg (nTreg). As used herein, the term "natural Treg" means a thymus-derived Treg. Natural T regs are CD4⁺CD25⁺FOXP3⁺Helios⁺Neuropilin 1⁺. Compared with iTregs, nTregs have higher expression of PD-1 (programmed cell death-1, pdcd1), Neuropilin 1 (Nrp1), Helios (Ikzf2), and CD73. nTregs may be distinguished from iTregs on the basis of the expression of Helios protein or Neuropilin 1 (Nrp1) individually.

The Treg may have a demethylated Treg-specific demethylated region (TSDR). The TSDR is an important methylation-sensitive element regulating Foxp3 expression (Polansky, J.K., et al., 2008. European journal of immunology, 38(6), pp.1654-1663).

Further suitable Tregs include, but are not limited to, Tr1 cells (which do not express Foxp3, and have high IL-10 production); CD8⁺FOXP3⁺ T cells; and γδ FOXP3⁺ T cells.

Different subpopulations of Tregs are known to exist, including naïve Tregs (CD45RA⁺FoxP3^{low}), effector/memory Tregs (CD45RA⁻FoxP3^{high}) and cytokine-producing Tregs (CD45RA⁻FoxP3^{low}). "Memory Tregs" are Tregs which express CD45RO and which are considered to be CD45RO⁺. These cells have increased levels of CD45RO as compared to naïve Tregs (e.g. at least 10, 20, 30, 40, 50, 60, 70, 80 or 90% more CD45RO) and which preferably do not express or have low levels of CD45RA (mRNA and/or protein) as compared to naïve Tregs (e.g. at least 80, 90 or 95% less CD45RA as compared to naïve Tregs). "Cytokine-producing Tregs" are Tregs which do not express or have very low levels of CD45RA (mRNA and/or protein) as compared to naïve Tregs (e.g. at least 80, 90 or 95% less CD45RA as compared to naïve Tregs), and which have low levels of FOXP3 as compared to Memory Tregs, e.g. less than 50, 60, 70, 80 or 90% of the FOXP3 as compared to Memory Tregs. Cytokine-producing Tregs may produce interferon gamma and may be less suppressive in vitro as compared to naïve Tregs (e.g. less than 50, 60, 70, 80 or 90% suppressive than naïve Tregs).

Reference to expression levels herein may refer to mRNA or protein expression. Particularly, for cell surface markers such as CD45RA, CD25, CD4, CD45RO, CD62L, etc., expression may refer to cell surface expression, i.e. the amount or relative amount of a marker protein that is expressed on the cell surface. Expression levels may be determined by any known method of the art. For example, mRNA expression levels may be determined by Northern blotting/array analysis, and protein expression may be determined by Western blotting, or preferably by FACS using antibody staining for cell surface expression.

Particularly, the Treg may be a naïve Treg. "A naïve regulatory T cell, a naïve T regulatory cell, or a naïve Treg" as used interchangeably herein refers to a Treg cell which expresses CD45RA (particularly which expresses CD45RA on the cell surface). Naïve Tregs are thus described as CD45RA⁺. Naïve Tregs generally represent Tregs which have not been activated through their endogenous TCRs by peptide/MHC, whereas effector/memory Tregs relate to Tregs which have been activated by stimulation through their endogenous TCRs. Typically, a naïve Treg may express at least 10, 20, 30, 40, 50, 60, 70, 80 or 90% more CD45RA than a Treg cell which is not naïve (e.g. a memory Treg cell). Alternatively viewed, a naïve Treg cell may express at least 2, 3, 4, 5, 10, 50 or 100-fold the amount of CD45RA as compared to a non-naïve Treg cell (e.g. a memory Treg cell). The level of expression of CD45RA can be readily determined by methods of the art, e.g. by flow cytometry using commercially available antibodies. Typically, non-naïve Treg cells do not express CD45RA or low levels of CD45RA.

Particularly, naïve Tregs may not express CD45RO, and may be considered to be CD45RO⁻. Thus, naïve Tregs may express at least 10, 20, 30, 40, 50, 60, 70, 80 or 90% less CD45RO as compared to a memory Treg, or alternatively viewed at least 2, 3, 4, 5, 10, 50 or 100 fold less CD45RO than a memory Treg cell.

Although naïve Tregs express CD25 as discussed above, CD25 expression levels may be lower than expression levels in memory Tregs, depending on the origin of the naïve Tregs. For example, for naïve Tregs isolated from peripheral blood, expression levels of CD25 may be at least 10, 20, 30, 40, 50, 60, 70, 80 or 90% lower than memory Tregs. Such naïve Tregs may be considered to express intermediate to low levels of CD25. However, a skilled person will appreciate that naïve Tregs isolated from cord blood may not show this difference.

Typically, a naïve Treg as defined herein may be CD4⁺, CD25⁺, FOXP3⁺, CD127^{low}, CD45RA⁺.

Low expression of CD127 as used herein refers to a lower level of expression of CD127 as compared to a CD4⁺ non-regulatory or Tcon cell from the same subject or donor. Particularly, naïve Tregs may express less than 90, 80, 70, 60, 50, 40, 30, 20 or 10% CD127 as compared to a CD4⁺ non-regulatory or Tcon cell from the same subject or donor. Levels of CD127 can be assessed by methods standard in the art, including by flow cytometry of cells stained with an anti-CD127 antibody.

Typically, naïve Tregs do not express, or express low levels of CCR4, HLA-DR, CXCR3 and/or CCR6. Particularly, naïve Tregs may express lower levels of CCR4, HLA-DR, CXCR3 and CCR6 than memory Tregs, e.g. at least 10, 20, 30, 40, 50, 60, 70, 80 or 90% lower level of expression.

Naïve Tregs may further express additional markers, including CCR7⁺ and CD31⁺ Isolated naïve Tregs may be identified by methods known in the art, including by determining the presence or absence of a panel of any one or more of the markers discussed above, on the cell surface of the isolated cells. For example, CD45RA, CD4, CD25 and CD127 low can be used to determine whether a cell is a naïve Treg. Methods of determining whether isolated cells are naïve Tregs or have a desired phenotype can be carried out as discussed below in relation to additional steps which may be carried out, and methods for determining the presence and/or levels of expression of cell markers are well-known in the art and include, for example, flow cytometry, using commercially available antibodies.

Suitably, the Treg or Treg population is isolated from a sample, wherein the sample comprises or consists of whole blood, umbilical cord blood, leukocyte cones, blood cones, peripheral blood mononuclear cells (PBMCs) or one or more leukopaks obtained from a subject. The Treg may be isolated from PBMCs obtained from a subject. The Treg may be isolated from leukocyte cones obtained from a subject. The Treg may be isolated from one or more leukopaks obtained from a subject. Suitably the subject from whom the sample is obtained is a mammal, preferably a human. Typically, a sample may be obtained by blood withdrawal or by apheresis.

Suitably the Treg is matched (e.g. HLA matched) or is autologous to the subject to whom the engineered Treg is to be administered. Suitably, the subject to be treated (i.e. to whom the engineered cell is to be administered) is a mammal, preferably a human. Allogeneic Tregs may be used where, for example, the cells have been subjected to gene editing techniques to prevent rejection or GvHD. The Treg cell may thus be generated *ex vivo* either from a patient's own peripheral blood (1st party), or in the setting of a haematopoietic stem cell transplant from donor peripheral blood (2nd party), or peripheral blood from an unconnected donor (3rd party).

Suitably, the Treg is part of a population of cells. Suitably, the population of Tregs comprises at least 60 % Tregs, such as at least 65, 70, 75, 80, 85, 90, 95, 97, 98 or 99 % Tregs. In one embodiment, the population of Tregs may comprise only Tregs. Such a population may be referred to as a "Treg population" or an "enriched Treg population". The terms "Treg population" and "a population of Tregs" are used interchangeably herein. Methods of obtaining or isolating a population of Tregs from a sample are discussed below. It will be appreciated by a skilled person that a population of Tregs may comprise other cell types which do not have a regulatory phenotype. Further, it is possible that the percentage of Tregs may increase in a Treg population during the methods of the invention due to the introduction of a polynucleotide encoding FOXP3. A Treg population may comprise different subpopulations of Tregs, or may comprise a single subpopulation of Treg, e.g. naïve Tregs.

In some embodiments, to provide a Treg population with high purity, the isolation/enrichment step of the methods of the present invention involves the depletion of CD8+ cells. Depletion of cells expressing CD8 relates to decreasing the percentage or number of such cells in the population but may not result in a complete removal of all cells expressing CD8. In some embodiments, at least 50%, at least 60%, at least 70%, at least 80%, at least 85%, at least 90%, at least 95% or 100% of CD8 cells may be depleted from the Treg population. CD8 depletion can be achieved using CD8 beads in line with standard protocols.

In some aspects, the Treg may be derived from *ex-vivo* differentiation of inducible progenitor cells (e.g. iPSCs) or embryonic progenitor cells to the Treg (e.g. differentiation directly to a Treg or indirectly through differentiation to a Tcon cell and conversion to a Treg as discussed below). A nucleic acid molecule or vector as described herein may be introduced into the inducible progenitor cells or embryonic progenitor cells prior to, or after, differentiation to a Treg. Suitable methods for differentiation are known in the art and include that disclosed in Haque et al, J Vis Exp., 2016, 117, 54720 (incorporated herein by reference).

As used herein, the term "conventional T cell" or Tcon or Tconv (used interchangeably herein) means a T lymphocyte cell which expresses an αβ T cell receptor (TCR) as well as a co-receptor which may be cluster of differentiation 4 (CD4) or cluster of differentiation 8 (CD8) and which does not have an immunosuppressive function. Conventional T cells are present in the peripheral blood, lymph nodes, and tissues. Suitably, the engineered Treg may be generated from a Tcon by introducing the nucleic acid which includes a sequence coding for FOXP3. Alternatively, the engineered Treg may be generated from a Tcon by *in vitro* culture of CD4+CD25-FOXP3- cells in the presence of IL-2 and TGF-β.

The invention provides a Treg or a Treg population comprising a Treg as defined or described herein. A "Treg" as used herein may also be referred to as a "cell" or a "Treg cell" and a "Treg population" as used herein may also be referred to as a "cell population" or a "Treg cell population". It will be appreciated that a Treg population may comprise both Treg cells of the invention comprising a nucleic acid molecule/polynucleotide molecule, polypeptide molecule, expression construct or vector as defined herein, and Treg cells which do not comprise a nucleic acid molecule/polynucleotide molecule, polypeptide molecule, expression construct or vector as described herein, e.g. untransduced or untransfected cells. Although in a preferred embodiment, all the Treg cells in a population may comprise a nucleic acid molecule/polynucleotide molecule, polypeptide molecule, expression construct or vector as described herein, cell populations having at least 10, 20, 30, 40, 50, 60, 70, 80, 90, 95 or 99% of cells comprising a nucleic acid molecule/polynucleotide molecule, polypeptide molecule, expression construct or vector of the invention are provided.

Natural Killer (NK) cells are immune cells which have been reported to have a regulatory effect. Particularly, NK cells which are CD56bright, may exhibit regulatory features. In this respect, the invention provides for the use of a NK cell or NK cell population in any method of the invention (i.e. the substitution of a Treg cell or Treg population with a NK cell or NK population). A NK cell or population is also provided which comprises an exogenous polynucleotide encoding a FOXP3 polypeptide, wherein the NK cell or population has higher CD62L expression after cryopreservation than a corresponding non-engineered NK cell or population after cryopreservation

### FORKHEAD BOX P3 PROTEIN (FOXP3)

In the present invention, FOXP3 expression is increased in Tregs by introducing into the cells a polynucleotide (sometimes referred to herein as a first polynucleotide) encoding a FOXP3 polypeptide.

"FOXP3" is the abbreviated name of the forkhead box P3 protein. FOXP3 is a member of the FOX protein family of transcription factors and functions as a master regulator of the regulatory pathway in the development and function of regulatory T cells. "FOXP3" as used herein encompasses variants, isoforms, and functional fragments of FOXP3.

The levels of FOXP3 mRNA and/or protein in a cell (or population of cells) may be increased in comparison to a corresponding cell (or population of cells) which has not been modified. For example, the level of FOXP3 mRNA and/or protein in a cell modified according to the present invention (or a population of such cells) may be increased to at least 1.5-fold, at least 2-fold, at least 5-fold, at least 10-fold, at least 50-fold, at least 100-fold, at least 150-fold greater than the level in a corresponding cell (or population of such cells) which has not been modified according to the present invention. The cell is a Treg or the population of cells is a population of Tregs.

Suitably, the level of FOXP3 mRNA and/or protein in a cell modified by according to the present invention (or a population of such cells) may be increased to at least 1.5-fold greater, 2-fold greater, or 5-fold greater than the level in a corresponding cell (or population of such cells) which has not been modified according to the present invention. The cell is a Treg or the population of cells is a population of Tregs.

Techniques for measuring the levels of specific mRNA and protein are well known in the art. mRNA levels in a population of cells, such as Tregs, may be measured by techniques such as the Affymetrix ebioscience prime flow RNA assay, Northern blotting, serial analysis of gene expression (SAGE) or quantitative polymerase chain reaction (qPCR). Protein levels in a population of cells may be measured by techniques such as flow cytometry, high-performance liquid chromatography (HPLC), liquid chromatography-mass spectrometry (LC/MS), Western blotting or enzyme-linked immunosorbent assay (ELISA).

A "FOXP3 polypeptide" is a polypeptide having FOXP3 activity i.e. a polypeptide able to bind FOXP3 target DNA and function as a transcription factor regulating development and function of Tregs. Particularly, a FOXP3 polypeptide may have the same or similar activity to wildtype FOXP3 (SEQ ID NO. 1), e.g. may have at least 40, 50, 60, 70, 80, 90, 95, 100, 110, 120, 130, 140 or 150% of the activity of the wildtype FOXP3 polypeptide. Techniques for measuring transcription factor activity are well known in the art. For example, transcription factor DNA-binding activity may be measured by ChIP. The transcription regulatory activity of a transcription factor may be measured by quantifying the level of expression of genes which it regulates. Gene expression may be quantified by measuring the levels of mRNA and/or protein produced from the gene using techniques such as Northern blotting, SAGE, qPCR, HPLC, LC/MS, Western blotting or ELISA. Genes regulated by FOXP3 include cytokines such as IL-2, IL-4 and IFN-γ (Siegler et al. Annu. Rev. Immunol. 2006, 24: 209-26, incorporated herein by reference). As discussed in detail below, FOXP3 or a FOXP3 polypeptide includes functional fragments, variants, and isoforms thereof, e.g. of SEQ ID NO. 1.

A "functional fragment of FOXP3" may refer to a portion or region of a FOXP3 polypeptide or a polynucleotide encoding a FOXP3 polypeptide that has the same of similar activity to the full-length FOXP3 polypeptide or polynucleotide. The functional fragment may have at least 40%, at least 50%, at least 60%, at least 70%, at least 80%, at least 90%, at least 95%, or 100% of the activity of the full-length FOXP3 polypeptide or polynucleotide. A person skilled in the art would be able to generate functional fragments based on the known structural and functional features of FOXP3. These are described, for instance, in Song, X., et al., 2012. Cell reports, 1(6), pp.665-675; Lopes, J.E., et al., 2006. The Journal of Immunology, 177(5), pp.3133-3142; and Lozano, T., et al, 2013. Frontiers in oncology, 3, p.294. Further, a N and C terminally truncated FOXP3 fragment is described within WO2019/241549 (incorporated herein by reference), for example, having the sequence SEQ ID NO. 5 as discussed below.

A "FOXP3 variant" may include an amino acid sequence or a nucleotide sequence which may be at least 50%, at least 55%, at least 65%, at least 70%, at least 75%, at least 80%, at least 85% or at least 90% identical, preferably at least 95% or at least 97% or at least 99% identical to a FOXP3 polypeptide or a polynucleotide encoding a FOXP3 polypeptide. FOXP3 variants may have the same or similar activity to a wildtype FOXP3 polypeptide or polynucleotide, e.g. may have at least 40, 50, 60, 70, 80, 90, 95, 100, 110, 120, 130, 140 or 150% of the activity of a wildtype FOXP3 polypeptide or polynucleotide. A person skilled in the art would be able to generate FOXP3 variants based on the known structural and functional features of FOXP3 and/or using conservative substitutions. FOXP3 variants may have similar or the same turnover time (or degradation rate) within a Treg cell as compared to wildtype FOXP3, e.g. at least 40, 50, 60, 70, 80, 90, 95, 99 or 100% of the turnover time (or degradation rate) of wildtype FOXP3 in a Treg. Some FOXP3 variants may have a reduced turnover time (or degradation rate) as compared to wildtype FOXP3, for example, FOXP3 variants having amino acid substitutions at amino acid 418 and/or 422 of SEQ ID NO. 1, for example S418E and/or S422A, as described in WO2019/241549 (incorporated herein by reference) and are set out in SEQ ID NO.s 2 to 4, which represent the aa418, aa422 and aa418 and aa422 mutants respectively.

Suitably, the FOXP3 polypeptide encoded by a nucleic acid molecule, construct or vector as described herein may comprise the polypeptide sequence of a human FOXP3, such as UniProtKB accession Q9BZS1 (SEQ ID NO: 1), or a functional fragment or variant thereof:
In some embodiments of the invention, the FOXP3 polypeptide comprises an amino acid sequence which is at least 70% identical to SEQ ID NO. 1 or a functional fragment thereof. Suitably, the FOXP3 polypeptide comprises an amino acid sequence which is at least 80%, at least 85%, at least 90%, at least 95%, at least 98% or at least 99% identical to SEQ ID NO. 1 or a functional fragment thereof. In some embodiments, the FOXP3 polypeptide comprises or consists of SEQ ID NO. 1 or a functional fragment thereof.

In some embodiments, as discussed above, the FOXP3 polypeptide may comprise mutations at residues 418 and/or 422 of SEQ ID NO. 1, as set out in SEQ ID NO. 2, SEQ ID NO. 3, or SEQ ID NO. 4.

In some embodiments of the invention, the FOXP3 polypeptide may be truncated at the N and/or C terminal ends, resulting in the production of a functional fragment. Particularly, an N and C terminally truncated functional fragment of FOXP3 may comprise or consist of an amino acid sequence of SEQ ID NO. 5 or a functional variant thereof having at least 80, 85, 90, 95 or 99% identity thereto.

Suitably, the FOXP3 polypeptide may be a variant of SEQ ID NO. 1, for example a natural variant. Suitably, the FOXP3 polypeptide is an isoform of SEQ ID NO. 1. For example, the FOXP3 polypeptide may comprise a deletion of amino acid positions 72-106 relative to SEQ ID NO. 1. Alternatively, the FOXP3 polypeptide may comprise a deletion of amino acid positions 246-272 relative to SEQ ID NO. 1.

Suitably, the FOXP3 polypeptide comprises SEQ ID NO. 6 or a functional fragment thereof. SEQ ID NO. 6 represents an illustrative FOXP3 polypeptide.

Suitably the FOXP3 polypeptide comprises or consists of an amino acid sequence which is at least 70% identical to SEQ ID NO. 6 or a functional fragment thereof. Suitably, the FOXP3 polypeptide comprises an amino acid sequence which is at least 80%, at least 85%, at least 90%, at least 95%, at least 98% or at least 99% identical to SEQ ID NO. 6 or a functional fragment thereof. In some embodiments, the FOXP3 polypeptide comprises or consists of SEQ ID NO. 6 or a functional fragment thereof.

Suitably, the FOXP3 polypeptide may be a variant of SEQ ID NO. 6, for example a natural variant. Suitably, the FOXP3 polypeptide is an isoform of SEQ ID NO. 6 or a functional fragment thereof. For example, the FOXP3 polypeptide may comprise a deletion of amino acid positions 72-106 relative to SEQ ID NO. 6. Alternatively, the FOXP3 polypeptide may comprise a deletion of amino acid positions 246-272 relative to SEQ ID NO. 6.

Suitably, the polynucleotide encoding a FOXP3 polypeptide comprises or consists of a polynucleotide sequence set forth in SEQ ID NO. 7, which represents an illustrative FOXP3 nucleotide sequence.

In some embodiments of the invention, the polynucleotide encoding the FOXP3 polypeptide or variant comprises a polynucleotide sequence which is at least 70% identical to SEQ ID NO. 7 or a fragment thereof which encodes a functional FOXP3 polypeptide. Suitably, the polynucleotide encoding the FOXP3 polypeptide or variant comprises a polynucleotide sequence which is at least 80%, at least 85%, at least 90%, at least 95%, at least 98% or at least 99% identical to SEQ ID NO. 7 or a functional fragment thereof. In some embodiments of the invention, the polynucleotide encoding the FOXP3 polypeptide or variant comprises or consists of SEQ ID NO. 7 or a fragment thereof which encodes a functional FOXP3 polypeptide.

Suitably, the polynucleotide encoding a FOXP3 polypeptide comprises or consists of a polynucleotide sequence set forth in SEQ ID NO. 8, which represents another illustrative FOXP3 nucleotide.

In some embodiments of the invention, the polynucleotide encoding the FOXP3 polypeptide or variant comprises a polynucleotide sequence which is at least 70% identical to SEQ ID NO. 8 or a fragment thereof which encodes a functional FOXP3 polypeptide. Suitably, the polynucleotide encoding the FOXP3 polypeptide or variant comprises a polynucleotide sequence which is at least 80%, at least 85%, at least 90%, at least 95%, at least 98% or at least 99% identical to SEQ ID NO. 8 or a fragment thereof which encodes a functional FOXP3 polypeptide. In some embodiments of the invention, the polynucleotide encoding the FOXP3 polypeptide or variant comprises or consists of SEQ ID NO. 8 or a fragment thereof which encodes a functional FOXP3 polypeptide.

Suitably, the polynucleotide encoding the FOXP3 polypeptide or functional fragment or variant thereof may be codon optimised. Suitably, the polynucleotide encoding the FOXP3 polypeptide or functional fragment or variant thereof may be codon optimised for expression in a human cell.

The term "variant" as used herein includes any substitution of, variation of, modification of, replacement of, deletion of and/or addition of one (or more) amino acid residues from or to the sequence providing that the resultant protein or polypeptide retains the desired function. Alternatively viewed, the variants or derivatives referred to herein are functional variants or derivatives.

Typically, amino acid substitutions may be made, for example from 1, 2 or 3 to 10 or 20 substitutions provided that the modified sequence retains the required activity or ability. Amino acid substitutions may include the use of non-naturally occurring analogues.

Proteins or peptides may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to Table 1 below.

**Table 1**

| | | |
|---|---|---|
| **ALIPHATIC** | **Non-polar** | **GAP** |
| | | **ILV** |
| | **Polar - uncharged** | **CSTM** |
| | | **N Q** |
| | **Polar - charged** | **D E** |
| | | **K R** |
| **AROMATIC** | | **H F W Y** |

Identity comparisons can be conducted by eye or, more usually, with the aid of readily available sequence comparison programs. These commercially available computer programs can calculate percentage identity between two or more sequences.

Percentage sequence identity may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percentage homology/sequence identity therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62. Suitably, the percentage identity is determined across the entirety of the reference and/or the query sequence. Once the software has produced an optimal alignment, it is possible to calculate percentage homology, preferably percentage sequence identity. The software typically does this as part of the sequence comparison and generates a numerical result.

Variants, and fragments may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

A skilled person will appreciate that FOXP3 expression within a Treg may be increased indirectly by introducing a polynucleotide into the cell which encodes a protein which increases transcription and/or translation of FOXP3 or which increases the half life (e.g. by at least 10, 20, 30, 40, 50, 60, 70, 80 or 90%) or function of FOXP3 (e.g. determined by suppressive ability of a transduced Treg, measured as previously discussed). For example, it may be possible to introduce a polynucleotide into a Treg which increases transcription of endogenous FOXP3 by interacting with the endogenous FOXP3 promoter or non-coding sequences (CNS, e.g. CNS1, 2 or 3) which are found upstream of the coding region. Particularly, FOXP3 expression may be increased within a Treg by introducing a polynucleotide encoding any one or more of c-Rel, p65, Smad3 or CREB.

In this aspect, the invention also provides a method of preserving CD62L expression in a regulatory T cell (Treg) population that has been cryopreserved, comprising introducing a polynucleotide encoding a protein which increases transcription and/or translation of FOXP3 or which increases the half-life or function of FOXP3.

Alternatively viewed, the invention also provides method of preserving CD62L expression in a regulatory T cell (Treg) population or Treg for cryopreservation comprising the steps of (a) introducing a polynucleotide encoding a protein which increases transcription and/or translation of FOXP3 or which increases the half-life or function of FOXP3 into the Treg population or Treg and (b) cryopreserving said Treg population or Treg.

Alternatively, the invention may provide a method for cryopreservation of a Treg (or a population of Tregs) comprising (a) introducing a polynucleotide encoding a protein which increases transcription and/or translation of FOXP3 or which increases the half-life or function of FOXP3 into a Treg or Treg population and (b) cryopreserving said Treg or Treg population, wherein said cryopreserved Treg or Treg population has a higher expression level of CD62L as compared to a corresponding non-engineered Treg or Treg population.

In another aspect, the invention provides a method for producing a cryopreserved Treg or population of Tregs having a level of CD62L comparable to a non-cryopreserved Treg or population of Tregs comprising (a) introducing a polynucleotide encoding a protein which increases transcription and/or translation of FOXP3 or which increases the half-life or function of FOXP3 into a Treg or population of Tregs and (b) cryopreserving said Treg or population of Tregs.

A skilled person will also appreciate that introduction of a polynucleotide into a cell as discussed herein (e.g. a Treg) which encodes CD62L directly may preserve the function (e.g. suppressive function) or stability after cryopreservation. In this aspect, the invention further provides a method of preserving function or stability of a Treg or Treg population (or other cell, e.g. NK cell) during/after cryopreservation comprising the step of introducing a polynucleotide encoding CD62L into the cell or cell population prior to cryopreservation. The invention further provides a cryopreserved cell (particularly Treg), comprising a polynucleotide encoding exogenous CD62L wherein said cell has comparable function or stability to a corresponding non-cryopreserved cell. The polynucleotide may encode CD62L comprising an amino acid sequence of SEQ ID NO. 9 or a functional fragment or functional variant thereof (i.e. a fragment or variant which retains at least 50, 60, 70, 80, 90 or 95% of the function of CD62L of SEQ ID NO. 9). Variants may have at least 60, 70, 80, 90 or 95% identity to SEQ ID NO. 9.

### PHENOTYPIC MARKER EXPRESSION

CD62L also known as L-selectin is a homing receptor or cell surface adhesion molecule, present on the extracellular surface of certain T lymphocytes (e.g. Tregs) and certain other immune cells, such as neutrophils. It belongs to the selectin family of proteins, which recognize sialylated carbohydrate groups. It is cleaved by ADAM17.

CD62L plays an important role in lymphocyte-endothelial cell interactions and CD62L expression has been linked to a more suppressive population following Treg expansion. CD62L acts as a "homing receptor" for Tregs to enter secondary lymphoid tissues via high endothelial venules. Ligands present on endothelial cells will bind to Tregs expressing CD62L, slowing Treg trafficking through the blood, and facilitating entry into a secondary lymphoid organ at that point.

The term "homing" or "home" as used herein means to travel to a site or location that contains a target, e.g. a binding target. In the context of a Treg cell expressing CD62L, this may mean to travel to a location where the CD62L binding partner MADCAM1 is expressed.

CD62L is a cell surface marker and the presence of CD62L, among other markers, is an indication that expanded cells have retained a Treg phenotype. Cells that have retained a Treg phenotype are also likely to have retained their usual suppressive function.

| |
|---|
| CD62L typically has an amino acid sequence of |
|  (SEQ ID NO. 9) |

A cryopreserved engineered Treg (or Treg population) as described herein may have preserved cell surface marker expression, particularly preserved CD62L cell surface expression, as compared to a cryopreserved Treg without exogenous FOXP3. "Preserved", "preserving" or "preservation" as used herein means that the amount of cell surface marker expression (e.g. CD62L) remains similar or comparable to the amount of cell surface marker expression on a corresponding fresh (i.e. a non-cryopreserved or non-frozen) Treg, i.e. cell surface marker expression may be the same as (i.e. not significantly different to) the amount of cell surface marker expression on a fresh Treg or it may be only 1% less, up to a maximum of 30% less, than the expression on a fresh Treg. Preferably, it will be no more than 5% less, 10% less, 15% less, 20% less or 25% less. It will be appreciated that typically, the cryopreserved Treg and fresh Treg cells used for comparison of expression levels of a cell surface marker (typically CD62L) will be the same cell type, or derived from the same cell type. For example, the levels of CD62L in a frozen naïve CD45RA+ Treg should be compared to the levels of CD62L in a fresh or non-frozen naïve CD56RA+ Treg. Typically, a corresponding non-cryopreserved cell (Treg) will have been processed or treated in the same way as a cell of the invention or used in the invention, and the same polynucleotide sequence will have been introduced into the corresponding non-cryopreserved cell. However, the cryopreservation step (and any step of thawing) will not have been carried out. Thus a corresponding non-cryopreserved cell, is typically a corresponding engineered non-cryopreserved cell.

Alternatively viewed, "preserved", "preserving" or "preservation" as used herein means that the amount of cell surface marker on a cryopreserved engineered Treg as described herein is higher (particularly significantly higher) than the amount of cell surface marker on a corresponding non-transduced cryopreserved Treg. Preferably, the amount of cell surface marker expression will be at least 10% higher, at least 15% higher, at least 20% higher, at least 25% higher, at least 30% higher, at least 35% higher, at least 40% higher, at least 45% higher, at least 50% higher, at least 55% higher, at least 60% higher, at least 65% higher, at least 70% higher or at least 80% higher than cell surface marker expression on a non-transduced cryopreserved Treg. The term "preserved" or "preserving" as used herein is interchangeable with the term "maintained" or "maintaining".

The term "higher CD62L expression" as used herein means that a cryopreserved engineered Treg as described herein has increased (or elevated) levels of CD62L expression as compared to a cryopreserved Treg without exogenous FOXP3. Thus, reference to a corresponding non-engineered cell or Treg herein, typically refers to a cell or Treg which has been or will be subjected to the same cryopreservation step (and any thawing step) as the cell or Treg of the invention or used in the invention, but an exogenous polynucleotide encoding FOXP3 will not have been introduced to the cell.

A cryopreserved Treg or Treg population comprising the polynucleotide, polypeptide, expression construct or vector as defined herein (and thus which has preserved and/or higher levels of CD62L expression as defined herein) may have an increased suppressive activity as compared to a non-engineered cryopreserved Treg or Treg population as defined herein (e.g. an increased suppressive activity of at least 5, 10, 20, 30, 40, 50, 60, 70, 80 or 90%).

Expression of CD62L can be determined by any well-known method of the art, including by Flow cytometry. Antibodies for CD62L detection and expression level determination are commercially available, for example, Thermofisher CD62L (L-Selectin) monoclonal antibody (MEL-14 or DREG-56).

As described herein, a polynucleotide encoding a FOXP3 polypeptide is introduced into a Treg prior to cryopreservation. The term "prior to cryopreservation" means before the Treg is cryopreserved (i.e. before the Treg is frozen). Thus, a Treg as described herein is not subjected to a step of cryopreservation before introduction of a polynucleotide encoding a FOXP3 polypeptide. Typically, the polynucleotide will be introduced into the Treg after isolation and before expansion, both of which may take place before cryopreservation.

### CRYOPRESERVATION

The terms "cryopreservation", or "cryopreserving" as used herein mean to freeze the Tregs, or Treg populations under conditions where cells remain viable (e.g. during freezing and after any subsequent step of thawing). Viability of cells can be measured by any well-known method of the art, for example flow cytometry using a live/dead stain (e.g. LIVE/DEAD^{™} Fixable Near-IR - Dead Cell Stain (Thermofisher). Typically, at least 50%, 60%, 70%, 80%, 90% or 95% of cells will remain viable during and after cryopreservation. Viability thus refers to live cells. A skilled person will appreciate that cryopreservation may allow cells to remain viable due to the application of one or more conditions which can effectively stop cell death and which can maintain the structure of the cell (for example, the use of a particular temperature, freezing/thawing rate and/or cryopreservant). Such conditions are known in the art and are discussed below.

A "cryopreserved Treg", or "cryopreserved Treg population" refer to a Treg, or Treg population that has undergone cryopreservation under conditions where cells remain viable as defined above and is cryopreserved (e.g. in a cryopreserved or frozen state). As discussed previously, a "cryopreserved Treg" comprises exogenous FOXP3. This may also be known as a "cryopreserved therapeutic Treg or Treg population" or a "cryopreserved engineered Treg or Treg population".

"A Treg or Treg population that has been cryopreserved" refers to a Treg or Treg population that has under cryopreservation as above and maybe cryopreserved (e.g. in a cryopreserved state) or may no longer be cryopreserved, i.e. includes a Treg, or Treg population that is in a thawed state (i.e. was previously cryopreserved).

"After cryopreservation" as used herein means that the cryopreservation process has been carried out. Tregs or Treg population after cryopreservation may be in a cryopreserved state (e.g. in storage) or may be thawed or undergoing thawing.

A "non-cryopreserved Treg" or "non-cryopreserved Treg population" refers to a Treg or Treg population that has not been cryopreserved, i.e. has never been cryopreserved or frozen. Such a cell may alternatively be referred to as fresh.

"Freezing", "frozen" or "frozen state" has its usual meaning in the art and is the process, or is the result of the process, in which a liquid changes to a solid. To freeze a Treg, or Treg population means to reduce the temperature of said Treg, or population to a temperature at which its particles no longer have enough energy to overcome the force of attraction between them.

Typically, a cryopreserved engineered Treg or Treg population will be stored at -196°C. However, a cryopreserved engineered Treg or Treg population may be stored at any temperature that is below freezing (i.e. any temperature below 0°C), where cells (or a proportion of the cells) remain viable as discussed above, for example, any temperature from -50°C to -196°C, from -80°C to -196°C, or any temperature less than -55°C, less than - 60°C, less than -65°C, less than -70°C, less than -75°C, less than -80°C, less than -85°C, less than -90°C, less than -95°C, less than -100°C, less than -105°C, less than -110°C, less than -115°C, less than -120°C, less than -125°C, less than -130°C, less than -135°C, less than -140°C, less than -145°C, less than -150°C, less than -155°C, less than -160°C, less than -165°C, less than -170°C. A cryopreserved Treg or Treg population may be stored at any temperature below freezing to -196°C, such as below -175°C, below -180°C, below - 185°C or below -190°C. Preferably, a cryopreserved Treg or Treg population may be stored at -80°C, at temperatures below -130°C or at the vapor phase of liquid nitrogen at approximately -196°C. More preferably, a cryopreserved Treg or Treg population may be stored in liquid nitrogen.

In the cryopreservation step of the present invention, the Treg or Treg population, any one or more (e.g. all) reagents (e.g. cryopreservation media) and devices (e.g. controlled rate devices and freezers, of which there are many known in the art, e.g. CryoMed, ThermoFisher) to be used may be pre-chilled (or pre-cooled) at the start of the cryopreservation process. "Pre-chilled" or "pre-chilling" as used herein means to reduce the temperature to between approximately 4°C to 8°C. This ensures that the temperature shock to the cells is minimised. The pre-chilling step may be, for example, approximately 5 minutes, 10 minutes, 15 minutes, 20 minutes or 30 minutes long.

During cryopreservation, the Treg population is typically protected by contacting with a cryopreservation media (also known as cryopreservation solution) comprising one or more cryoprotectants as described herein. This is usually carried out prior to lowering the temperature of the Treg or Treg population to the cryopreservation temperature, i.e. to the temperature at which the Treg or Treg population will be stored. The Treg population may be centrifuged to form a cell pellet, and resuspended in cryopreservation media prior to reducing the temperature.

Cryopreservation media typically comprises a complete growth media, which may be specifically optimised to provide viable cells on thawing, together with a cryoprotectant, as discussed above. Many different types of cryopreservation media are commercially available, for example, ImmunoCult- XF T cell medium (StemCell). Preferably, the cryopreservation media may be any media that conforms to GMP standards. Cryoprotectants are substances used to protect biological tissue from freezing damage caused by the formation of ice crystals. Cryoprotectants fall into two general categories; permeating cryoprotectants, which can pass through cell membranes, and non-permeating cryoprotectants, any of which can be used in accordance with the cryopreservation step of a method of the invention. Examples of permeating cryoprotectants include, but are not limited to, dimethyl sulfoxide (DMSO), glycerol, sucrose and 1,2-propanediol. Examples of non-permeating cryoprotectants include, but are not limited to, hydroxyethyl starch, albumin, and polyvinyl pyrrolidone. The most used permeative cryoprotectant is DMSO, which is often used in combination with a non-permeative agent such as autologous plasma, serum albumin, and/or hydroxyethyl starch. Any cryoprotectant known in the art may be used in the method of the present invention. Preferably, the cryoprotectant used in the method of the present invention may be any cryoprotectant that conforms to Good Manufacturing Process (GMP) standards.

A skilled person will appreciate that the number of Tregs which may be resuspended in cryopreservation media (i.e. media containing one or more cryoprotectants) may depend on the dose to be provided to a subject, which will vary between Treg products and disease or condition to be treated. A Treg population may be cryopreserved at any desired cell density.

The optimal rate of freezing the Treg or Treg population may be determined by several factors, including the permeability of the Treg cell membrane to water, the cell surface to volume ratio and the type and concentration of the cryoprotectant used. The Treg or Treg population may be continuously cooled between about 4°C and -80°C and particularly, a controlled rate of freezing may be used (e.g. of approximately -1°C per minute). Alternatively, the controlled rate of freezing may be, for example, approximately -2°C per minute, -3°C per minute, -4°C per minute or -5°C per minute. To achieve controlled rate freezing, any suitable controlled rate freezing device known in the art may be used. A skilled person will understand that ice nucleation (i.e. inducing the population to freeze by ice crystal formation in the extracellular fluid) may be initiated by any suitable means at approximately -5°C. Once the temperature reaches approximately -80°C, the Treg or population can be transferred directly into liquid nitrogen (-196°C) for storage.

The Treg or Treg population may be stored at -80°C for any desirable length of time, for example any time from approximately 4 to 48 hours, preferably for 4 to 16 hours or for up to 24 hours, and then may be transferred to liquid nitrogen storage at temperatures below - 130°C. Alternatively, once the Treg or Treg population reaches -80°C, it may be transferred immediately to liquid nitrogen storage at temperatures below -130°C. Storage in liquid nitrogen may be for any desirable amount of time. Storage may be for any number of days or weeks. Typically, storage may be long-term, i.e. for any number of months, for a year or for any number of years, for example 2 years, 5 years, 10 years, 20 years, 50 years, or 100 years.

Alternatively, the Treg or Treg population may be cryopreserved by vitrification. Vitrification is a very rapid method of cryopreservation whereby the Treg or Treg population may be transitioned from approximately 37°C to -196°C in a short period of time (typically under 1 second), resulting in extremely fast rates of cooling.

The Treg or Treg population may be thawed after cryopreservation. "Thawing", "thawed" or "thawed state" has its usual meaning in the art and is the process, or is the result of the process, in which a solid changes to a liquid. To thaw a Treg, or Treg population means to increase the temperature of said Treg, or population to a temperature at which its particles have enough energy to overcome the force of attraction between them. Thus, in order to thaw the Treg or Treg population, heat is usually applied. Typically, thawed cells will be capable of expansion and/or of functioning as Tregs (e.g. will be capable of eliciting their immunosuppressive function). A thawed Treg or Treg population therefore refers to a Treg or population which has been subjected to both cryopreservation and a thawing process. A skilled person will appreciate that a thawed Treg population or the process of thawing may not result in the complete thawing of the population and that a portion of cells within the population may remain frozen. For example, a thawed Treg population may comprise at least 10, 20, 30, 40 or 50% frozen or cryopreserved cells. Alternatively viewed, a thawed Treg population may comprise no more than 50, 40, 30, 20 or 10% frozen or cryopreserved cells.

The optimal rate of thawing varies depending on the cryopreservation conditions used. In general, rapid heating may be applied for cryopreserved Treg cells, which can be accomplished by placing or by agitating (e.g. by shaking) the composition in a water bath at a temperature of approximately 37°C. The Treg or Treg population may be warmed to chilled temperatures (approximately 0-10°C), until a small ice crystal remains or may be warmed to human body temperature of approximately 37°C e.g. for administration directly to a patient.

In one embodiment, the cryopreserved Treg or Treg population may be warmed to a temperature at which it changes from a solid to a liquid and be administered directly to a patient.

The Treg or Treg population of the invention can be administered parenterally, for example, intravenously, or by infusion techniques. Parenteral administration as used herein means modes of administration other than enteral and topical administration, usually by injection, and includes intravenous, intramuscular, intraarterial, intrathecal, intracapsular, intraorbital, intracardiac, intraarticular, transtracheal, intradermal, intraperitoneal, subcutaneous, subcuticular, subcapsular, subarachnoid, intraspinal and intrasternal injection and infusion.

### T CELL RECEPTORS (TCRs) AND CHIMERIC ANTIGEN RECEPTORS (CARs)

Suitably, the Treg or Treg population of the present invention may also comprise an exogenous TCR or a CAR.

A TCR is a cell surface molecule that binds fragments of antigen bound to major histocompatibility complex (MHC) molecules on antigen presenting cells as part of directing an immune response. Suitably, the TCR may be a recombinant protein, in other words the TCR may be an exogenous protein which is not naturally expressed by the present Treg of the invention.

The terms "TCR" and "CAR (chimeric antigen receptor)" as used herein refer to engineered receptors which can confer an antigen specificity onto Treg cells (for example Tregs).

CARs are also known as artificial T-cell receptors, chimeric T-cell receptors or chimeric immunoreceptors. A CAR typically comprises an extracellular domain comprising an antigen-specific targeting region, termed herein an antigen-binding domain, a transmembrane domain, and an endodomain comprising optionally one or more co-stimulatory domains, and an intracellular signaling domain. The antigen-binding domain is typically joined to the transmembrane domain by a hinge domain. The design of CARs, and the various domains that they may contain, is well known in the art.

The antigen-binding domain of a CAR may be derived or obtained from any protein or polypeptide which binds (i.e. has affinity for) a desired target antigen, or more generally a desired target molecule. This may be for example, a ligand or receptor, or a physiological binding protein for the target molecule, or a part thereof, or a synthetic or derivative protein. The target molecule may commonly be expressed on the surface of a cell, for example a target cell, or a cell in the vicinity of a target cell (for a bystander effect), but need not be. Depending on the nature and specificity of the antigen binding domain, the CAR may recognise a soluble molecule, for example where the antigen-binding domain is based on, or derived from, a cellular receptor.

The antigen-binding domain is most commonly derived from antibody variable chains (for example it commonly takes the form of a scFv), but may also be generated from T-cell receptor variable domains or, as mentioned above, other molecules, such as receptors for ligands or other binding molecules.

The CAR is typically expressed as a polypeptide also comprising a signal sequence (also known as a leader sequence), and in particular a signal sequence which targets the CAR to the plasma membrane of the cell. This will generally be positioned next to or close to the antigen-binding domain, generally upstream of the antigen-binding domain. The extracellular domain, or ectodomain, of the CAR may thus comprise a signal sequence and an antigen-binding domain.

The antigen-binding domain provides the CAR with the ability to bind a predetermined antigen of interest. The antigen-binding domain preferably targets an antigen of clinical interest or an antigen at a site of disease.

As noted above, the antigen-binding domain may be any protein or peptide that possesses the ability to specifically recognize and bind to a biological molecule (e.g., a cell surface receptor or a component thereof). The antigen-binding domain includes any naturally occurring, synthetic, semi-synthetic, or recombinantly produced binding partner for a biological molecule of interest. Illustrative antigen-specific targeting domains include antibodies or antibody fragments or derivatives, extracellular domains of receptors, ligands for cell surface molecules/receptors, or receptor binding domains thereof, and tumor binding proteins. Although as discussed below, the antigen-specific targeting domain may preferably be an antibody or derived from an antibody, other antigen-specific targeting domains are encompassed, e.g. antigen-specific targeting domains formed from an antigenic peptide/MHC or HLA combination which is capable of binding to the TCRs of Tcon cells active at a site of transplantation, inflammation or disease.

The antigen binding domain may be an antibody, or derived from, an antibody. An antibody-derived binding domain can be a fragment of an antibody or a genetically engineered product of one or more fragments of the antibody, which fragment is involved in binding with the antigen. Examples include a variable region (Fv), a complementarity determining region (CDR), Fab or F(ab')₂, or the light and heavy chain variable regions can be joined together in a single chain (e.g. as a ScFv) and in either orientation (e.g. V_{L}-V_{H} or V_{H}-V_{L}). The V_{L} and/or V_{H} sequences may be modified. In particular the framework regions may be modified (e.g. substituted, for example to humanise the antigen-binding domain). Other examples include a heavy chain variable region (VH), a light chain variable region (VL) a camelid antibody (VHH) and a single domain antibody (sAb).

Typically, the binding domain is a single chain antibody (scFv). The scFv may be murine, human or humanized scFv.

"Complementarity determining region" or "CDR" with regard to an antibody or antigen-binding fragment thereof refers to a highly variable loop in the variable region of the heavy chain or the light chain of an antibody. CDRs can interact with the antigen conformation and largely determine binding to the antigen (although some framework regions are known to be involved in binding). The heavy chain variable region and the light chain variable region each contain 3 CDRs. "Heavy chain variable region" or "VH" refers to the fragment of the heavy chain of an antibody that contains three CDRs interposed between flanking stretches known as framework regions, which are more highly conserved than the CDRs and form a scaffold to support the CDRs. "Light chain variable region" or "VL" refers to the fragment of the light chain of an antibody that contains three CDRs interposed between framework regions. "Fv" refers to the smallest fragment of an antibody to bear the complete antigen binding site. An Fv fragment consists of the variable region of a single light chain bound to the variable region of a single heavy chain. "Single-chain Fv antibody" or "scFv" refers to an engineered antibody consisting of a light chain variable region and a heavy chain variable region connected to one another, in either orientation, directly or via a peptide linker sequence. Antibodies that specifically bind a predetermined antigen can be prepared using methods well known in the art. Such methods include phage display, methods to generate human or humanized antibodies, or methods using a transgenic animal or plant engineered to produce human antibodies. Phage display libraries of partially or fully synthetic antibodies are available and can be screened for an antibody or fragment thereof that can bind to the target molecule. Phage display libraries of human antibodies are also available. Once identified, the amino acid sequence or polynucleotide sequence coding for the antibody can be isolated and/or determined.

The CAR may be directed towards any desired target antigen or molecule. This may be selected according to the intended therapy, and the condition it is desired to treat. It may for example be an antigen or molecule associated with a particular condition, or an antigen or molecule associated with a cell it is desired to target to treat the condition. Typically, the antigen or molecule is a cell-surface antigen or molecule.

The term "directed against" is synonymous with "specific for" or "anti". Put another way, the CAR recognises a target molecule. Accordingly, it is meant that the CAR is capable of binding specifically to a specified or given antigen, or target. **In** particular, the antigen-binding domain of the CAR is capable of binding specifically to the target molecule or antigen (more particularly when the CAR is expressed on the surface of a cell, notably an immune effector cell). Specific binding may be distinguished from non-specific binding to a non-target molecule or antigen. Thus, a cell expressing the CAR is directed, or re-directed, to bind specifically to a target cell, expressing the target molecule or antigen, particularly a target cell expressing the target antigen or molecule on its cell surface.

Antigens which may be targeted by a CAR include, but are not limited to, antigens expressed on cells associated with transplanted organs, autoimmune diseases, allergic diseases, metabolic diseases (e.g. diabetes mellitus) and inflammatory diseases (e.g. neurodegenerative disease).

Antigens associated with organ transplants and/or cells associated with transplanted organs include, but are not limited to, a HLA antigen present in the transplanted organ but not in the patient, or an antigen whose expression is up-regulated during transplant rejection such as CCL19, MMP9, SLC1A3, MMP7, HMMR, TOP2A, GPNMB, PLA2G7, CXCL9, FABP5, GBP2, CD74, CXCL10, UBD, CD27, CD48, CXCL11.

In an embodiment the CAR is directed against an HLA antigen, and in particular an HLA-A2 antigen.

Antibodies against such antigens are known in the art, and conveniently a scFv may be obtained or generated based on a known or available antibody. In this regard VH and VL, and CDR sequences are publicly available to aid the preparation of such an antibody-binding domain, for example in WO 2020/044055, the disclosure of which is herein incorporated by reference. Any of the antigen binding domains, or CDR, VH, and/or VL sequences disclosed in WO 2020/044055 may be used.

In an embodiment, the antigen-binding domain comprises VH CDR1, 2 and 3 sequences as set forth in SEQ ID NOs. 10, 11 and 12 respectively and VL CDR1, 2 and 3 sequences as set forth in SEQ ID NOs. 13, 14 and 15 respectively.

Where a CDR does contain an amino acid sequence modification, this may be a deletion, addition, or substitution of an amino acid residue of the CDR sequence as set out in the above-mentioned SEQ ID NOs. More particularly, the modification may be an amino acid substitution, for example a conservative amino acid substitution, e.g. as set out above. A longer CDR may tolerate more amino acid residue modifications. In the case of CDRs which are 5 or 7 amino acid residues long, the modifications may be to or of 1 or 2, e.g. 1, residue. In general, there may be 0, 1, 2, or 3 modifications to any particular CDR sequence. Further, in an embodiment, CDRs 1 and 2 may be modified, and CDR3 may be unmodified. In another embodiment all 3 CDRs may be modified. In another embodiment, the CDRs are not modified.

The antigen binding domain may be in the form of a scFV comprising the VH and VL domain sequences as set out above, in either order, for example VH-VL. The VH and VL sequences may be linked by a linker sequence.

Suitable linkers can be readily selected and can be of any of a suitable length, such as from 1 amino acid (e.g. Gly) to 30 amino acids, e.g. from any one of 2, 3, 4, 5, 6, 7,8, 9, or 10 amino acids to any one of 12, 15, 18, 20, 21, 25, 30 amino acids, for example, 5-30, 5-25, 6-25, 10-15, 12-25, 15 to 25 etc.

The CAR also preferably comprises a hinge domain to hold the extracelluar domain, particularly the antigen binding domain, away from the cell surface, and comprises a transmembrane domain. The hinge and transmembrane domains may comprise the hinge and transmembrane sequences from any protein which has a hinge domain and/or a transmembrane domain, including any of the type I, type II or type III transmembrane proteins.

The transmembrane domain of the CAR may also comprise an artificial hydrophobic sequence. The transmembrane domains of the CAR may be selected so as not to dimerize. Additional transmembrane domains will be apparent to those of skill in the art. Examples of transmembrane (TM) regions used in CAR constructs are: 1) The CD28 TM region (Pule et al, Mol Ther, 2005, Nov;12(5):933-41; Brentjens et al, CCR, 2007, Sep 15;13(18 Pt 1):5426-35; Casucci et al, Blood, 2013, Nov 14;122(20):3461-72.); 2) The OX40 TM region (Pule et al, Mol Ther, 2005, Nov;12(5):933-41); 3) The 41BB TM region (Brentjens et al, CCR, 2007, Sep 15;13(18 Pt 1):5426-35); 4). The CD3 zeta TM region (Pule et al, Mol Ther, 2005, Nov;12(5):933-41; Savoldo B, Blood, 2009, Jun 18;113(25):6392-402.); 5) The CD8a TM region (Maher et al, Nat Biotechnol, 2002, Jan;20(1):70-5.; Imai C, Leukemia, 2004, Apr;18(4):676-84; Brentjens et al, CCR, 2007, Sep 15;13(18 Pt 1):5426-35; Milone et al, Mol Ther, 2009, Aug;17(8):1453-64.). Other transmembrane domains which may be used include those from CD4, CD45, CD9, CD16, CD22, CD33, CD64, CD80, CD86, or CD154.

A hinge domain may conveniently be obtained from the same protein as the transmembrane domain, although this is not essential.

Alternatively, the CAR may comprise a domain derived from the CD8α transmembrane domain.

The endodomain of a CAR as described herein comprises motifs necessary to transduce the effector function signal and direct a cell expressing the CAR to perform its specialized function upon antigen binding. Particularly, the endodomain may comprise one or more (e.g. two or three) Immunoreceptor tyrosine-based activation motifs (ITAMs), typically comprising the amino acid sequence of YXXL/I, where X can be any amino acid. Examples of intracellular signaling domains include, but are not limited to, ζ chain endodomain of the T-cell receptor or any of its homologs (e.g., η chain, FcεR1γ and β chains, MB1 (Igα) chain, B29 (Igβ) chain, etc.), CD3 polypeptide domains (Δ, δ and ε), syk family tyrosine kinases (Syk, ZAP 70, etc.), src family tyrosine kinases (Lck, Fyn, Lyn, etc.) and other molecules involved in T-cell transduction, such as CD2, CD5 and CD28. The intracellular signaling domain may comprise human CD3 zeta chain endodomain, FcyRIII, FcsRI, cytoplasmic tails of Fc receptors, immunoreceptor tyrosine-based activation motif (ITAM) bearing cytoplasmic receptors or combinations thereof.

Other signaling domains which may be used include the signaling domains of CD28 or CD27 or variants thereof. Additional intracellular signaling domains will be apparent to those of skill in the art and may be used in connection with alternate embodiments of the invention.

The CAR may comprise a compound endodomain comprising a fusion of the intracellular part of a T-cell co-stimulatory molecule to that of e.g. CD3ζ. Such a compound endodomain may be referred to as a second-generation CAR which can transmit an activating and co-stimulatory signal simultaneously after antigen recognition. The co-stimulatory domain most commonly used is that of CD28. This supplies the most potent co-stimulatory signal - namely immunological signal 2, which triggers T-cell proliferation. The CAR endodomain may also comprise one or more TNF receptor family signalling domains, such as the signalling domain of OX40, 4-1BB, ICOS or TNFRSF25.

### SAFETY SWITCH

The safety switch polypeptide provides a cell in or on which it is expressed with a suicide moiety. This is useful as a safety mechanism which allows a cryopreserved cell which has been administered to a subject to be deleted should the need arise, or indeed more generally, according to desire or need, for example once a cell has performed or completed its therapeutic effect. As discussed above, a Treg of the invention or used in the invention may additionally comprise a safety switch polypeptide.

A suicide moiety possesses an inducible capacity to lead to cellular death, or more generally to elimination or deletion of a cell. An example of a suicide moiety is a suicide protein, encoded by a suicide gene, which may be expressed in or on a Treg as described herein. A suicide moiety herein is a suicide polypeptide, that is a polypeptide that under permissive conditions, namely conditions that are induced or turned on, is able to cause the cell to be deleted.

The suicide moiety may be a polypeptide, or amino acid sequence, which may be activated to perform a cell-deleting activity by an activating agent which is administered to the subject, or which is active to perform a cell-deleting activity in the presence of a substrate which may be administered to a subject. In a particular embodiment, the suicide moiety may represent a target for a separate cell-deleting agent which is administered to the subject. By binding to the suicide moiety, the cell-deleting agent may be targeted to the cell to be deleted. In particular, the suicide moiety may be recognised by an antibody, and binding of the antibody to the safety switch polypeptide, when expressed on the surface of a cell, causes the cell to be eliminated, or deleted.

The suicide moiety may be HSV-TK or iCasp9. However, it is preferred for the suicide moiety to be, or to comprise, an epitope which is recognised by a cell-deleting antibody or other binding molecule capable of eliciting deletion of the cell. In such an embodiment the safety switch polypeptide is expressed on the surface of a cell.

In particular, the suicide moiety may be a CD20 epitope which is recognised by the antibody Rituximab. Thus, in the safety switch polypeptide the suicide moiety may comprise a minimal epitope based on the epitope from CD20 that is recognised by the antibody Rituximab. Cryopreserved cells expressing a safety switch polypeptide comprising this sequence can be selectively killed using the antibody Rituximab, or an antibody having the binding specificity of Rituximab. The safety switch polypeptide is expressed on the cell surface and when the expressed polypeptide is exposed to or contacted with Rituximab, or an antibody with the same binding specificity, death of the cell ensues.

For example, the suicide constructs of WO2013/153391 (incorporated herein by reference) may be used in a Treg or Treg population as described herein.

### POLYNUCLEOTIDE

Nucleic acid molecules and polynucleotides/nucleic acid sequences as defined herein and used interchangeably herein, may comprise DNA or RNA. They may be single-stranded or double-stranded. It will be understood by a skilled person that numerous different nucleic acid molecules/polynucleotides can encode the same polypeptide as a result of the degeneracy of the genetic code.

The nucleic acid molecules/polynucleotides may be modified by any method available in the art. Such modifications may be carried out in order to enhance the in vivo activity or lifespan of the nucleic acid molecules/polynucleotides as defined herein.

Nucleic acid molecules/polynucleotides/nucleotide sequences such as DNA nucleic acid molecules/polynucleotides/sequences may be produced recombinantly, synthetically or by any means available to those of skill in the art. They may also be cloned by standard techniques.

Longer nucleic acid molecules/polynucleotides/nucleotide sequences will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

The nucleic acid molecule/polynucleotide described herein may further comprise a nucleic acid sequence encoding a selectable marker. Suitably selectable markers are well known in the art and include, but are not limited to, fluorescent proteins - such as GFP. Suitably, the selectable marker may be a fluorescent protein, for example GFP, YFP, RFP, tdTomato, dsRed, or variants thereof. In some embodiments the fluorescent protein is GFP or a GFP variant. The nucleic acid sequence encoding a selectable marker may be provided in combination with a nucleic acid molecule herein in the form of a nucleic acid construct. Such a nucleic acid construct may be provided in a vector.

Suitably, the selectable marker/reporter domain may be a luciferase-based reporter, a PET reporter (e.g. Sodium Iodide Symporter (NIS)), or a membrane protein (e.g. CD34, low-affinity nerve growth factor receptor (LNGFR)).

The nucleic acid sequences encoding FOXP3 and one or more other polypeptides, e.g. a CAR, safety switch and/or selectable markers, when comprised within a single construct may be separated from each other, by one or more co-expression sites which enables expression of each polypeptide as a discrete entity. Suitable co-expression sites are known in the art and include, for example, internal ribosome entry sites (IRES) and self-cleaving sites such as those included in the present nucleic acid molecules, and as defined below. In an embodiment this may be a 2A cleavage site, as discussed below.

Suitable self-cleaving domains include P2A, T2A, E2A, and F2A sequences

The use of a selectable marker is advantageous as it allows cells (e.g. Tregs) in which a nucleic acid molecule, construct or vector has been successfully introduced (such that the encoded FOXP3 polypeptide and possibly CAR and safety switch polypeptides are expressed) to be selected and isolated from a starting cell population using common methods, e.g. flow cytometry.

Expression of a polynucleotide as described herein, e.g. encoding FOXP3 will typically be controlled by a promoter. A "promoter" is a region of DNA that leads to initiation of transcription of a gene. Promoters are located near the transcription start sites of genes, upstream on the DNA (towards the 5' region of the sense strand). Any suitable promoter may be used, the selection of which may be readily made by the skilled person. The promoter may be from any source, and may be a viral promoter, or a eukaryotic promoter, including mammalian or human promoters (i.e. a physiological promoter). In an embodiment the promoter is a viral promoter. Particular promoters include LTR promoters, EFS (or functional truncations thereof), SFFV, PGK, and CMV. In an embodiment the promoter is SFFV or a viral LTR promoter. "Operably linked to the same promoter" means that transcription of the polynucleotide sequences may be initiated from the same promoter (e.g. transcription of the first, second and third polynucleotide sequences is initiated from the same promoter) and that the nucleotide sequences are positioned and oriented for transcription to be initiated from the promoter. Polynucleotides operably linked to a promoter are under transcriptional regulation of the promoter.

### VECTOR

In some embodiments of the invention, the polynucleotide is within an expression vector. The term "expression vector" as used herein means a construct enabling expression of the FOXP3 polypeptide (or another polypeptide resulting in preserved levels of CD62L).

A vector is a tool that allows or facilitates the transfer of an entity from one environment to another. As used herein, and by way of example, some vectors used in recombinant nucleic acid techniques allow entities, such as a segment of nucleic acid (e.g. a heterologous DNA segment, such as a heterologous cDNA segment), to be transferred into a target cell.

Vectors may be non-viral or viral. Examples of vectors used in recombinant nucleic acid techniques include, but are not limited to, plasmids, mRNA molecules (e.g. in vitro transcribed mRNAs), chromosomes, artificial chromosomes and viruses. The vector may also be, for example, a naked nucleic acid (e.g. DNA). In its simplest form, the vector may itself be a nucleotide of interest.

The vectors used herein may be, for example, plasmid, mRNA or virus vectors and may include a promoter (as described below) for the expression of a nucleic acid molecule/polynucleotide and optionally a regulator of the promoter. The term "nucleic acid molecule" as used herein includes polynucleotides.

In an embodiment the vector is a viral vector, for example a retroviral, e.g. a lentiviral vector or a gamma retroviral vector.

The vectors may further comprise additional promoters, for example, in one embodiment, the promoter may be a LTR, for example, a retroviral LTR or a lentiviral LTR. Long terminal repeats (LTRs) are identical sequences of DNA that repeat hundreds or thousands of times found at either end of retrotransposons or proviral DNA formed by reverse transcription of retroviral RNA. They are used by viruses to insert their genetic material into the host genomes. Signals of gene expression are found in LTRs: enhancer, promoter (can have both transcriptional enhancers or regulatory elements), transcription initiation (such as capping), transcription terminator and polyadenylation signal. Suitably, the vector may include a 5'LTR and a 3'LTR.

The vector may comprise one or more additional regulatory sequences which may act pre- or post-transcriptionally. "Regulatory sequences" are any sequences which facilitate expression of the polypeptides, e.g. act to increase expression of a transcript or to enhance mRNA stability. Suitable regulatory sequences include for example enhancer elements, post-transcriptional regulatory elements and polyadenylation sites. Suitably, the additional regulatory sequences may be present in the LTR(s). Suitably, the vector may comprise a Woodchuck Hepatitis Virus Posttranscriptional Regulatory Element (WPRE), e.g. operably linked to the promoter.

Vectors comprising a nucleic acid molecule/polynucleotide as described herein may be introduced into cells using a variety of techniques known in the art, such as transformation and transduction. Several techniques are known in the art, for example infection with recombinant viral vectors, such as retroviral, lentiviral, adenoviral, adeno-associated viral, baculoviral and herpes simplex viral vectors; direct injection of nucleic acids and biolistic transformation.

Non-viral delivery systems include but are not limited to DNA transfection methods. Here, transfection includes a process using a non-viral vector to deliver a gene to a target cell. Non-viral delivery systems can include liposomal or amphipathic cell penetrating peptides, preferably complexed with a nucleic acid molecule or construct.

Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nat. Biotechnol. (1996) 14: 556) and combinations thereof.

Engineered Treg cells may be generated by introducing a nucleic acid molecule, construct, or vector as defined herein, by one of many means including transduction with a viral vector, and transfection with DNA or RNA.

A Treg cell as defined herein may be made by: introducing to a cell (e.g. by transduction or transfection) the nucleic acid molecule/polynucleotide, construct or vector as defined herein.

Suitable cells are discussed further above, but the cell may be from a sample isolated from a subject. The subject may be a donor subject, or a subject for therapy (i.e. the cell may be an autologous cell, or a donor cell, for introduction to another recipient, e.g. an allogeneic cell).

The cell may be generated by a method comprising the following steps:
(i) isolation of a Treg cell-containing sample from a subject or provision of a Treg cell-containing sample; and
(ii) introduction into (e.g. by transduction or transfection) the Treg cell-containing sample of a nucleic acid molecule, construct, or vector as defined herein, to provide a population of engineered Treg cells.

A target Treg cell-enriched sample may be isolated from, enriched, and/or generated from the cell-containing sample prior to and/or after step (ii) of the method. For example, isolation, enrichment and/or generation of Tregs (or other target cells) may be performed prior to and/or after step (ii) to isolate, enrich or generate a Treg-enriched sample. Isolation and/or enrichment from a cell-containing sample may be performed after step (ii) to enrich for Tregs (or other target cells) comprising the nucleic acid molecule/polynucleotide, the construct and/or the vector encoding FOXP3 as described herein.

A Treg-enriched sample may be isolated or enriched by any method known to those of skill in the art, for example by FACS and/or magnetic bead sorting. A Treg-enriched sample may be generated from the cell-containing sample by any method known to those of skill in the art, for example, from Tcon cells by introducing DNA or RNA coding for FOXP3 and/or from ex-vivo differentiation of inducible progenitor cells or embryonic progenitor cells. Methods for isolating and/or enriching other target cells are known in the art.

Suitably, an engineered target cell may be generated by a method comprising the following steps:
(i) isolation of a target-cell enriched sample from a subject or provision of a target cell-enriched sample; and
(ii) introduction into (e.g. by transduction or transfection) the target cell-enriched sample of a nucleic acid, construct or vector as defined herein, to provide a population of engineered target cells.

The target cell may be a Treg cell, or precursor or a progenitor therefor. Isolation of a Treg cell may comprise the following steps:
(i) isolating CD4+ T cells; and
(ii) isolating the Treg from the CD4+ T cells.

The isolation may comprise selecting said T cells or Treg cells using immune-magnetic beads or fluorescence-activated cell sorting (FACS).

An "engineered cell", "engineered Treg" or "engineered Treg population" means a cell or cell population which has been modified to comprise or express a polynucleotide which is not naturally present within the cell. A skilled person will appreciate that whilst Tregs express FOXP3, an engineered cell as described herein will comprise a non-naturally occurring polynucleotide sequence encoding FOXP3 (e.g. comprising non-naturally occurring promoters, regulatory sequences, CAR, TCR etc). Methods for engineering cells are known in the art and include, but are not limited to, genetic modification of cells e.g. by transduction such as retroviral or lentiviral transduction, transfection (such as transient transfection - DNA or RNA based) including lipofection, polyethylene glycol, calcium phosphate and electroporation, as discussed above. Any suitable method may be used to introduce a nucleic acid sequence into a cell. Non-viral technologies such as amphipathic cell penetrating peptides may be used to introduce a nucleic acid.

A non-engineered Treg or cell is one which does not contain an exogenous polynucleotide/nucleic acid molecule encoding FOXP3. Thus, the non-engineered Treg or cell may comprise other exogenous polynucleotides (i.e. other than FOXP3). In one embodiment, the non-engineered Treg or cells does not contain any exogenous polynucleotide/nucleic acid molecule.

Suitably, an engineered cell is a cell which has been modified e.g. by transduction or by transfection. Suitably, an engineered cell is a cell which has been modified or whose genome has been modified e.g. by transduction or by transfection. Suitably, an engineered cell is a cell which has been modified or whose genome has been modified by retroviral transduction. Suitably, an engineered cell is a cell which has been modified or whose genome has been modified by lentiviral transduction.

As used herein, the term "introduced" refers to methods for inserting foreign nucleic acid, e.g. DNA or RNA, into a cell. As used herein the term introduced includes both transduction and transfection methods. Transfection is the process of introducing nucleic acids into a cell by non-viral methods. Transduction is the process of introducing foreign DNA or RNA into a cell via a viral vector. Engineered cells may be generated by introducing a nucleic acid as described herein by one of many means including transduction with a viral vector, transfection with DNA or RNA.

Cells may be activated and/or expanded prior to, or after, the introduction of a nucleic acid/polynucleotide as described herein, for example by treatment with an anti-CD3 monoclonal antibody or both anti-CD3 and anti-CD28 monoclonal antibodies. The cells may also be expanded in the presence of anti-CD3 and anti-CD28 monoclonal antibodies in combination with IL-2. Suitably, IL-2 may be substituted with IL-15. Other components which may be used in a cell (e.g. Treg) expansion protocol include, but are not limited to rapamycin, all-trans retinoic acid (ATRA) and TGFβ. As used herein "activated" means that a cell has been stimulated, causing the cell to proliferate. As used herein "expanded" means that a cell or population of cells has been induced to proliferate. The expansion of a population of cells may be measured for example by counting the number of cells present in a population. The phenotype of the cells may be determined by methods known in the art such as flow cytometry.

### COMPOSITIONS

A pharmaceutical composition is a composition that comprises or consists of a therapeutically effective amount of a pharmaceutically active agent i.e. the Treg, or Treg population. It preferably includes a pharmaceutically acceptable carrier, diluent or excipient (including combinations thereof). Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s) or solubilising agent(s). It will be understood that the pharmaceutical composition of the invention may be a cryopreserved pharmaceutical composition.

By "pharmaceutically acceptable" is included that the formulation is sterile and pyrogen free. The carrier, diluent, and/or excipient must be "acceptable" in the sense of being compatible with the cell or vector and not deleterious to the recipients thereof. Typically, the carriers, diluents, and excipients will be saline or infusion media which will be sterile and pyrogen free, however, other acceptable carriers, diluents, and excipients may be used.

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

The Treg, Treg populations or pharmaceutical compositions described herein may be administered in a manner appropriate for treating and/or preventing the desired disease or condition and suitable routes of administration are discussed herein. The quantity and frequency of administration will be determined by such factors as the condition of the subject, and the type and severity of the subject's disease or condition, although appropriate dosages may be determined by clinical trials. The pharmaceutical composition may be formulated accordingly.

The Treg, Treg population or pharmaceutical composition as described herein can be administered parenterally, for example, intravenously, or they may be administered by infusion techniques. The Treg, Treg population or pharmaceutical composition may be administered in the form of a sterile aqueous solution which may contain other substances, for example, enough salts or glucose to make the solution isotonic with blood. The aqueous solution may be suitably buffered (preferably to a pH of from 3 to 9). The pharmaceutical composition may be formulated accordingly. The preparation of suitable parenteral formulations under sterile conditions is readily accomplished by standard pharmaceutical techniques well-known to those skilled in the art.

The pharmaceutical compositions may comprise cells in infusion media, for example sterile isotonic solution. The pharmaceutical composition may be enclosed in ampoules, disposable syringes or multiple dose vials made of glass or plastic.

The Treg, Treg population or pharmaceutical composition may be administered in a single or in multiple doses. Particularly, the Treg, Treg population or pharmaceutical composition may be administered in a single, one-off dose. The pharmaceutical composition may be formulated accordingly.

The pharmaceutical composition may further comprise one or more active agents. The pharmaceutical composition may further comprise one or more other therapeutic agents, such as lympho-depletive agents (e.g. thymoglobulin, campath-1H, anti-CD2 antibodies, anti-CD3 antibodies, anti-CD20 antibodies, cyclophosphamide, fludarabine), inhibitors of mTOR (e.g. sirolimus, everolimus), drugs inhibiting costimulatory pathways (e.g. anti-CD40/CD40L, CTAL4Ig), and/or drugs inhibiting specific cytokines (IL-6, IL-17, TNFalpha, IL18).

Depending upon the disease/condition and subject to be treated, as well as the route of administration, the Treg, Treg population or pharmaceutical composition may be administered at varying doses (e.g. measured in cells/kg or cells/subject). The physician in any event will determine the actual dosage which will be most suitable for any individual subject and it will vary with the age, weight and response of the particular subject. Typically, however, for the cells herein, doses of 5x10⁷ to 3x10⁹ cells, or 1x10⁸ to 2x10⁹ cells per subject may be administered.

The Treg, Treg populations and pharmaceutical compositions herein may be for use in treating or preventing a disease or condition. The cells and compositions containing them are for adoptive cell therapy (ACT). Various conditions may be treated by administration of Treg cells, for example, Treg cells expressing a TCR or CAR. As noted above, these may be conditions responsive to immunosuppression, and particularly the immunosuppressive effects of Treg cells. The cells, cell populations and pharmaceutical compositions described herein may thus be used for inducing, or achieving, immunosuppression in a subject. The Treg cells administered, or modified in vivo, may be targeted by expression of the TCR or CAR. Conditions suitable for such treatment include infectious, neurodegenerative, inflammatory, or metabolic diseases, or more broadly a condition associated with any undesired or unwanted or deleterious immune response.

In particular, the Treg cells, and Treg populations, and compositions provide a means for inducing tolerance to a transplant; treating and/or preventing cellular and/or humoral transplant rejection; treating and/or preventing graft-versus-host disease (GvHD), an autoimmune or allergic disease; a neurodegenerative disease such as amyloid lateral sclerosis (ALS); a metabolic disease such as type I diabetes; or to promote tissue repair and/or tissue regeneration; or to ameliorate inflammation/suppress an immune response. The cells, cell populations, and compositions may be used in a method which comprises the step of administering a cell, cell population, or a composition as described herein to a subject.

As used herein, "inducing tolerance to a transplant" refers to inducing tolerance to a transplanted organ in a recipient. In other words, inducing tolerance to a transplant means to reduce the level of a recipient's immune response to a donor transplant organ. Inducing tolerance to a transplanted organ may reduce the amount of immunosuppressive drugs that a transplant recipient requires, or may enable the discontinuation of immunosuppressive drugs. In this regard, the invention further provides a method for inducing tolerance to a transplanted organ in a subject, comprising administering a Treg, population or composition as discussed herein to the subject.

For example, the Tregs, may be administered to a subject with a disease in order to lessen, reduce, or improve at least one symptom of disease such as jaundice, dark urine, itching, abdominal swelling or tenderness, fatigue, nausea or vomiting, and/or loss of appetite. The at least one symptom may be lessened, reduced, or improved by at least 10%, at least 20%, at least 30%, at least 40%, or at least 50%, or the at least one symptom may be completely alleviated.

The Tregs may be administered to a subject with a disease in order to slow down, reduce, or block the progression of the disease. The progression of the disease may be slowed down, reduced, or blocked by at least 10%, at least 20%, at least 30%, at least 40%, or at least 50% compared to a subject in which the engineered cells are not administered, or progression of the disease may be completely stopped.

**In** one embodiment, the subject is a transplant recipient undergoing immunosuppression therapy.

Suitably, the subject is a mammal. Suitably, the subject is a human.

The transplant may be selected from a liver, kidney, heart, lung, pancreas, intestine, stomach, bone marrow, vascularized composite tissue graft, and skin transplant.

The Treg, Treg population or pharmaceutical composition of the invention may be administered to a patient immediately after/during thawing and without further expansion. The Treg, Treg population or pharmaceutical composition may be administered to a patient within about 5, 10, 15, or 30 minutes, or about 1, 2, 3, 4, 5, 6, 7, 8, 9, 10, 11 or 12 hours of thawing.

A method for treating a disease or condition relates to the therapeutic use of the cells herein. In this respect, the cells may be administered to a subject having an existing disease or condition in order to lessen, reduce or improve at least one symptom associated with the disease or condition and/or to slow down, reduce or block the progression of the disease. Suitably, treating and/or preventing cellular and/or humoral transplant rejection may refer to administering an effective amount of the cells (e.g. Tregs) such that the amount of immunosuppressive drugs that a transplant recipient requires is reduced, or may enable the discontinuation of immunosuppressive drugs.

Preventing a disease or condition relates to the prophylactic use of the cells herein. In this respect, the cells may be administered to a subject who has not yet contracted or developed the disease or condition and/or who is not showing any symptoms of the disease or condition to prevent the disease or condition or to reduce or prevent development of at least one symptom associated with the disease or condition. The subject may have a predisposition for, or be thought to be at risk of developing, the disease or condition.

The autoimmune or allergic disease may be selected from inflammatory skin diseases including psoriasis and dermatitis (e.g. atopic dermatitis); responses associated with inflammatory bowel disease (such as Crohn's disease and ulcerative colitis); dermatitis; allergic conditions such as food allergy, eczema and asthma; rheumatoid arthritis; systemic lupus erythematosus (SLE) (including lupus nephritis, cutaneous lupus); diabetes mellitus (e.g. type 1 diabetes mellitus or insulin dependent diabetes mellitus); multiple sclerosis; neurodegenerative disease, for example, Amyotrophic Lateral Sclerosis (ALS); Chronic inflammatory demyelinating polyneuropathy (CIPD) and juvenile onset diabetes.

In some embodiments, the disease or disorder to be treated is a neurological/neurodegenerative disease or disorder/condition. In some embodiments, the neurological/neurodegenerative disease or disorder is associated with inflammation. Thus, in some embodiments, the invention may find utility in treating or preventing (e.g. reducing the risk of) neuroinflammation or an associated disease or disorder. The neuroinflammation may be chronic or acute, preferably chronic. The neuroinflammation may be neuroinflammation of the central or peripheral nervous system, preferably the central nervous system.

In some embodiments the neurological disease, disorder or injury is selected from amyotrophic lateral sclerosis (ALS), dementia, frontotemporal dementia, Alzheimer's disease, vascular dementia, mixed dementia, Creutzfeldt-Jakob disease, Chronic Inflammatory Demyelinating Polyneuropathy (CIDP), Huntington's disease, Tauopathy disease, Nasu-Hakola disease, central nervous system lupus, Parkinson's disease, dementia with Lewy bodies, Multiple System Atrophy (Shy-Drager syndrome), progressive supranuclear palsy, cortical basal ganglionic degeneration, acute disseminated encephalomyelitis, seizures, spinal cord injury, traumatic brain injury (e.g. ischemia and traumatic brain injury), depression, autism spectrum disorder and multiple sclerosis. In some embodiments, the neurological disease, disorder, or injury is amyotrophic lateral sclerosis (ALS), Alzheimer disease, Parkinson disease, multiple sclerosis, ischemia and traumatic brain injury, depression, and autism spectrum disorder.

Amyotrophic lateral sclerosis (ALS) (also known as motor neuron disease or Lou Gehrig's disease) refers to a debilitating disease with varied etiology characterized by rapidly progressive weakness, muscle atrophy and fasciculations, muscle spasticity, difficulty speaking (dysarthria), difficulty swallowing (dysphagia), and difficulty breathing (dyspnea).

Thus, the invention provides a Treg, Treg population or pharmaceutical composition as defined herein for use in therapy (e.g. cryopreserved, thawed or Treg population obtainable by a method of the invention).

Suitably, the Treg may be autologous. Suitably, the Treg may be allogenic.

Suitably, the Treg (e.g. the engineered Treg) may be administered in combination with one or more other therapeutic agents, such as lympho-depletive agents (e.g. as discussed above).

The Treg, may be administered simultaneously with or sequentially with (i.e. prior to or after) the one or more other therapeutic agents.

Tregs, may be activated and/or expanded prior to, or after, the introduction of a nucleic acid molecule as described herein, for example by treatment with an anti-CD3 monoclonal antibody or both anti-CD3 and anti-CD28 monoclonal antibodies. Expansion protocols are discussed above. Preferably, the activation and/or expansion process occurs prior to cryopreservation. Preferably, no further activation and/or expansion is required after thawing.

The Tregs, may be washed after each step of the method, in particular after expansion.

The population of Treg cells may be further enriched by any method known to those of skill in the art, for example by FACS or magnetic bead sorting.

The steps of the method of production and cryopreservation may be performed in a closed and sterile cell culture system.

As discussed previously, the invention further provides a method of improving the homing ability of a Treg or Treg population to secondary lymphoid organs after cryopreservation as compared to a corresponding non-engineered Treg or Treg population after cryopreservation, comprising the step of introducing a polynucleotide encoding a FOXP3 polypeptide into the Treg or Treg population prior to cryopreservation.

"Improving the homing ability" of a Treg or Treg population refers to an increased ability to home or locate to secondary lymphoid organs after in vivo administration (e.g. an increase of at least 10, 20, 30, 40, 50, 60, 70, 80 or 90%) as compared to a Treg or Treg population which do not comprise an exogenous polynucleotide encoding FOXP3. Homing ability can be measured by any known imaging techniques.

Further, the invention provides a method for increasing the stability and/or suppressive function of a Treg or Treg population after cryopreservation comprising the step of introducing a nucleic acid molecule/polynucleotide, an expression construct or vector as provided herein into the cell prior to cryopreservation. Increasing the stability refers to an increase in stability of at least 10, 20, 30, 40, 50, 60, 70, 80 or 90% (e.g. as compared to a corresponding cryopreserved non-engineered Treg). Stability can be determined by measuring Treg numbers present over time, using methods known in the art. Increasing the suppressive function of a Treg refers to an increase in suppressive function of at least 10, 20, 30, 40, 50, 60, 70, 80 or 90% (e.g. as compared to a corresponding cryopreserved non-engineered Treg). Suppressive function can be determined by any well known assay, including that described herein. Alternatively viewed, the invention provides a method for preserving the stability and/or suppressive function of a Treg or Treg population after cryopreservation comprising the step of introducing a nucleic acid molecule/polynucleotide, an expression construct or vector as provided herein into the cell prior to cryopreservation, wherein the stability and/or suppression function is comparable to a corresponding non-cryopreserved engineered Treg. Comparable function/stability means that function or stability may be similar or the same to the corresponding non-cryopreserved engineered Treg, e.g. has at least 70, 80 or 90% of stability or function.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively. The correct nucleic acid and amino acid sequences come directly from the inventors.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of also include the term "consisting of'.

The publications discussed herein are provided solely for their disclosure prior to the filing date of the present application and are herein incorporated by reference. Nothing herein is to be construed as an admission that such publications constitute prior art to the claims appended hereto.

Various modifications and variations of the disclosed methods, cells, compositions and uses of the invention will be apparent to the skilled person without departing from the scope and spirit of the invention. Although the invention has been disclosed in connection with specific preferred embodiments, it should be understood that the invention as claimed should not be Funduly limited to such specific embodiments. Indeed, various modifications of the disclosed modes for carrying out the invention, which are obvious to the skilled person are intended to be within the scope of the following claims.

The invention will now be further described by way of Examples, which are meant to serve to assist one of ordinary skill in the art in carrying out the invention and are not intended in any way to limit the scope of the invention.

### EXAMPLES

### Materials and methods

### Cloning:

Construct 1 (C1) was designed in house, and the whole sequence was codon optimised for expression in human cells and manufactured. The construct was cloned into the pMP71 backbone and D5a high efficiency bacteria were transformed with plasmid and grown with the selection agent ampicillin. DNA was extracted using a Miniprep Kit (Qiagen). Inserts were transferred into a lentiviral backbone by PCR cloning. Construct 1 (C1) represents a construct of the present disclosure as claimed herein.

### Collection of PBMCs:

Leukocyte cones were supplied by NHS blood and transplant and leukopaks were obtained from BiolVT. PBMCs were isolated from cones using a density centrifugation protocol. Briefly, blood was diluted 1:1 with 1xPBS and layered over Ficoll-Paque (GE Healthcare). Samples were centrifuged and the leukocyte layer was removed and washed in PBS.

### Treg isolation protocol:

Blood cones and leukopaks were subjected to CD4 enrichment via negative selection using RosetteSep^{™} Human CD4+ T Cell Enrichment Cocktail. Subsequently, CD4+ cells were isolated using density centrifugation. CD4+ CD25+ T cells were then isolated via positive selection using CD25 microbeads II (Miltenyi). The CD4+CD25+ fraction was stained with flow cytometry antibodies CD4 FITC (OKT4, Biolegend), CD25 PE-Cy7 (BC96, Biolegend), CD127 BV421 (A019D5, Biolegend), CD45RA BV510 (HI100, Biolegend) and the LIVE/DEAD^{™} Fixable Near-IR - Dead Cell Stain (Thermofisher) before FACS sorting. CD4+CD25+CD127low CD45RA+ (CD45RA+ Tregs) were sorted and used.

### Treg culture media and expansion:

Human Regulatory T cells were activated with Human T-Activator CD3/CD28 Dynabeads^{™} (Gibco) and cultured in XVIVO (Lonza) supplemented with IL-2 and 5% of human serum (Sigma-Aldrich). Cells were re-fed every 2 to 3 days with Treg culture media. A second round of stimulation with Dynabeads^{™} was performed to promote further expansion of Treg cells.

### Transfection and viral particle production:

### Lentivirus:

HEK293T cells were seeded and cultured in DMEM (Dulbecco's Modified Eagle's Medium) + 10% Fetal Bovine Serum (FBS) for 24 hours. Transfection reagents were brought to room temperature and were mixed with DNA construct/plasmid of interest, packaging plasmid (pD8.91) and viral envelope (pVSV-G). PEI was added to the diluted DNA and mixed and added to HEK293Ts. Supernatant was harvested 48 hours post-transfection, filtered and virus concentrated.

### Transduction of T cells

Treg cells were activated with anti-CD3 and anti-CD28 Dynabeads (Gibco) and resuspended in T cell culture media. Non-tissue culture-treated 24-well plates were prepared by coating with Retronectin (Takahara-bio - Otsu, Japan) and cell suspension was added together with lentiviral supernatant. Cells were incubated and media exchange was performed on alternate days. Cells were used for experiments 12 days post-transduction.

### Flow cytometry staining to determine transduction and FOXP3 expression

Treg cells were removed from culture, then washed and stained with the LIVE/DEAD^{™} Fixable Near-IR - Dead Cell Stain (Thermofisher) in PBS first and then with anti-CD4 AF700 (RPA-T4, BD), anti-CD34 FITC (QBEND/10, Thermofisher) and anti-CD3 PE-Cy7 in FACS staining buffer. For intracellular staining of FOXP3, cells were fixed and permeabilized and stained with the anti-Foxp3 PE (150D/E4, Thermofisher) antibody. Cells were analysed on a Attune NxT flow cyotmeter.

### Flow cytometry phenotyping of transduced cells

Treg cells were removed from culture and stained for live cells using the dextramer and LIVE/DEAD^{™} Fixable Near-IR - Dead Cell Stain as described above. Surface staining of the cells was performed using anti-CD62L BV650 (Biolegend). Cells were permeabilised and stained with anti-Foxp3 PE (150D/E4, Thermofisher).

### Cryopreservation and thawing of human Treg cells

After 14 days of *in vitro* expansion, Dynabeads were magnetically removed from Treg cells and the cells were rested at 1x10^6/mL density in cell culture medium with 1% human serum. The following day, half of the cells were cryopreserved, and the other half were used fresh for phenotypic analysis. Cryopreservation was carried out by resuspending rested Treg cells in cryopreservation media to obtain 0.5-10x10⁶ cells/mL of cell suspension. Cells were then transferred into cryovials using a sterile Pasteur pipette and the cryovials allowed to freeze inside a pre-chilled freezing container for 4-6 hours at -80°C. Cryovials were finally transferred into a liquid nitrogen tank. Thawing of Treg cells was carried out by immersing cryovials into a water bath for 2-3 mins at 37°C. The thawed cell suspension was then gently poured into a conical tube containing cell culture medium with 20% fetal bovine serum (FBS) and the cells were centrifuged for 10 mins at 300xg. After centrifugation, cells were counted and used for phenotypic analysis.

### Methods for testing different cryopreservation solutions and cell densities

The in-house construct, Construct 1 (C1), was designed and manufactured using standard protocols. This construct represents a construct of the present disclosure as claimed herein.

Naïve Tregs were isolated from leukopaks and sorted according to standard protocols. The isolated cells were activated with CD3/CD28 beads and cultured in XVIVO (Lonza) supplemented with IL-2 and human serum. Cells were re-fed every 2 to 3 days with culture media. A second round of stimulation with CD3/CD28 beads was performed during culturing to promote further expansion of Treg cells.

Treg cells were either non-transduced or transduced during culture with C1 using lentiviral vector (lentiviral vector was produced according to standard protocols). Vector and IL-2 were added direct to the Treg cells in culture.

After expansion, a portion of cells were taken for counts and flow cytometry analysis ('Pre-freeze'). The remaining cells were aliquoted into 5 different cell volumes. The cells were spun down and the cell pellet was resuspended in 1ml of cryopreservation solution 1, 2 or 3, to give 5 different cell densities per cryopreservation solution (ranging from 1x to 100x the original cell density). The cells were then aliquoted into cryovials and placed into -80°C for 24 hours and then transferred to liquid nitrogen. Thawing of the Tregs was conducted by placing the cryovials in a 37°C water bath until a small ice crystal remained.

The cells were then analysed by flow cytometry to assess transduction, FOXP3 expression and phenotype. This was done using the in-house 'health' flow cytometry panel and the in-house 'maturation' flow cytometry panel. Briefly, the health panel measures live/dead, CD4/CD25, FOXP3, RQR8 (using an anti-CD34 antibody - indicates transduction) and apoptotic markers. The maturation panel measures live/dead, CD4, CD45RA (Naïve/SCM), CD45RA-CCR7+ (central memory), CD45RA-CCR7- (effector memory) and CD62L. Results are shown in Figures 4 to 6.

### Data analysis

Flow cytometric data was analysed using the Flow Cytometry Analysis software FlowJo (Flowjo,LLC). All statistical Analysis were performed using Graphpad Prism v.8 (Graphpad, Software)

### Example 1 - viability assessment

As described above, fresh and frozen non-transduced Tregs, i.e. non-genetically modified Tregs (Non-GMO) that do not contain exogenous FOXP3, and fresh and frozen Tregs transduced with a construct expressing FOXP3 (C1 (Construct 1)) were collected and stained for flow cytometry analysis with a fixable viability dye. Cells from eight separate donors were assessed. Construct 1 (C1) is a construct comprising FOXP3, a safety switch and a CD34 selectable marker. Detection of the CD34 selectable marker using an anti-CD34 antibody (such as QBEND) enables identification of transduced cells. C1 represents a construct of the present disclosure as claimed herein.

Figure 1 shows that the percentage viability of frozen transduced Tregs is comparable to the percentage viability of frozen non-transduced Tregs at approximately 75%.

### Example 2 - Transduction and FOXP3 expression

Transduction efficiency in fresh and frozen Tregs transduced with a construct expressing FOXP3 (C1) was assessed as described above. Transduced cells were selected using an anti-CD34 antibody. Cells from 6 separate donors were assessed.

Figure 2 shows that both fresh and frozen Tregs transduced with C1 expressed FOXP3.

### Example 3 - phenotype assessment (cell surface expression of CD62L)

Fresh and frozen non-transduced Tregs (Non-GMO), i.e. Tregs that do not contain exogenous FOXP3, and fresh and frozen Tregs transduced with an experimental construct expressing FOXP3 (C1 (Construct 1)) were collected and stained for flow cytometry analysis with an anti-CD62L antibody conjugated to a fluorophore as described above. Cells from eight separate donors were assessed.

Figure 2 shows that the level of CD62L expression remains close to 100% in Tregs transduced with FOXP3 and in non-transduced Tregs when these cells have not been frozen. CD62L expression levels decrease in both transduced and non-transduced cells after cryopreservation. Crucially, however, Tregs transduced with FOXP3 maintain significantly higher levels of CD62L expression after freezing and thawing as compared to non-transduced Tregs.

### Example 4 - viability assessment in different cryopreservation solutions

Tregs were either transduced with a construct expressing FOXP3 (C1 (Construct 1)) or were non-transduced, i.e. non-genetically modified Tregs (Non-GMO) that do not contain exogenous FOXP3. Some of these Tregs were used fresh for viability analysis ('pre-freeze' columns in Figure 4) and the others were frozen in three different cryopreservation solutions: Solution 1, Solution 2 and Solution 3 at five different cell densities per solution. The fresh and frozen Tregs were collected and stained for flow cytometry analysis with a viability dye, as described above. Cells transduced with C1 were selected using an anti-CD34 antibody. Cells from 5 separate donors were assessed.

Figure 4 shows that the percentage viability of frozen transduced and non-transduced Tregs is comparable to that of fresh transduced and non-transduced Tregs, at almost 100%, and that there is very little change in percentage viability across the three different cryopreservation solutions 1, 2 and 3, and across all five cell densities, in both transduced and non-transduced cells.

### Example 5 - Transduction and FOXP3 expression in different cryopreservation solutions

Transduction efficiency was assessed, as described above, in fresh Tregs that were either transduced with a construct expressing FOXP3 (C1) or non-transduced, i.e. non-genetically modified Tregs (Non-GMO) that do not contain exogenous FOXP3 (see 'pre-freeze' columns in Figure 5), and in frozen Tregs that were either transduced with C1 or non-transduced and that had been frozen in cryopreservation Solution 1, 2 or 3 at five different cell densities per solution. Cells transduced with C1 were selected using an anti-CD34 antibody. Cells from 5 separate donors were assessed.

Figure 5 shows that both fresh and frozen Tregs transduced with C1 expressed high levels of FOXP3 and that the percentage of FOXP3 expression was consistent across all three cryopreservation solutions, at all five cell densities.

### Example 6 - phenotype assessment (cell surface expression of CD62L) in different cryopreservation solutions

Tregs were either transduced with a construct expressing FOXP3 (C1 (Construct 1)) or were non-transduced, i.e. non-genetically modified Tregs (Non-GMO) that do not contain exogenous FOXP3. Some of these Tregs were used fresh for phenotypic analysis ('pre-freeze' columns in Figure 6) and the others were frozen in three different cryopreservation solutions: Solution 1, Solution 2 or Solution 3 at five different cell densities per solution. The fresh and frozen Tregs were collected and stained for flow cytometry analysis with an anti-CD62L antibody conjugated to a fluorophore, as described above. Cells from 5 separate donors were assessed.

Figure 6 shows that the level of CD62L expression remains close to 90% in Tregs transduced with FOXP3 (C1) across all three cryopreservation solutions, 1, 2 and 3, and at the five cell densities, whereas CD62L expression in the non-transduced Tregs is much lower in all conditions.

## Claims

1. A method of preparing a Treg population suitable for immediate administration to a subject, comprising the steps of (a) introducing a polynucleotide encoding a FOXP3 polypeptide into a Treg population, (b) cryopreserving said Treg population and (c) thawing said Treg population, wherein no further culture or expansion is carried out after step (c).

2. The method according to claim 1, further comprising a step of isolating a Treg population from a sample prior to introducing the polynucleotide.

3. The method according to claim 1 or 2, further comprising the step of expanding the Treg population prior to step (b).

4. The method according to claims 2 or 3, wherein the sample consists of whole blood, umbilical cord blood, leukocyte cones, blood cones, peripheral blood mononuclear cells (PBMCs) or one or more leukopaks.

5. The method according to any one of claims 1 to 4, wherein:
(i) step (b) comprises the following steps of:
(bi) suspending said Treg population in a cryopreservation media;
(bii) freezing the Treg population of (bi); and
(biii) storing the Treg population of (bii) at a temperature below -130°C,
optionally wherein the method further comprises the step of storing the Treg population at -80°C for up to 24 hours prior to step (biii); and/or
(ii) the method further comprises the steps of:
pre-chilling said Treg population, and/or any one or more reagents and devices to be used in the cryopreservation step, prior to step (b); and/or
cryopreserving said Treg population according to step (b) at a controlled rate of freezing of approximately -1°C per minute.

6. The method according to any of claims 1 to 5, wherein thawing the Treg population comprises warming the Treg population from a temperature below -130°C to a temperature of between approximately 0-10°C, optionally wherein warming the Treg population comprises placing the Treg population in a water bath maintained at approximately 37°C.

7. The method according to any of claims 2 to 6, wherein the Treg population is isolated by selecting for:
(a)
(i) CD4⁺CD25⁺CD127⁻ and/or CD4⁺CD25⁺CD127^{low} cells; or
(ii) CD4⁺CD25^{hi}CD127⁻ and/or CD4⁺CD25⁺CD127^{low} cells; and/or
(b) the Treg population is isolated by selecting for CD45RA⁺ cells, preferably CD4⁺CD25⁺CD127^{low}CD45RA⁺ cells.

8. The method according to any of claims 1 to 7, wherein the FOXP3 polypeptide comprises an amino acid sequence which is at least 80% identical to SEQ ID NO. 1 or a functional fragment thereof.

9. The method according to any of claims 1 to 8, wherein the polynucleotide encoding FOXP3 is within an expression vector.

10. The method according to any of claims 1 to 9, further comprising introducing a polynucleotide encoding an exogenous T cell receptor (TCR) or a polynucleotide encoding a chimeric antigen receptor (CAR) into the Treg population.

11. The method according to claim 10, wherein the polynucleotide encoding the FOXP3 polypeptide and the polynucleotide encoding the exogenous TCR or the CAR are provided by a single expression vector.

12. The method according to claim 11, wherein the vector comprises a first polynucleotide encoding the FOXP3 polypeptide and a second polynucleotide encoding the exogenous TCR or CAR, wherein the first polynucleotide and the second polynucleotide are operably linked to the same promoter, and wherein the first polynucleotide is upstream of the second polynucleotide.

13. The method according to any of claims 10 to 12, wherein there is an internal self-cleaving sequence between the polynucleotide encoding FOXP3 and the polynucleotide encoding the exogenous TCR or CAR.

14. A Treg population for use in prevention and/or treatment of a disease, wherein the Treg population is obtained by the method of any one of claims 1 to 13 and wherein the Treg population is for administration to a subject immediately after thawing.

15. A Treg population for use according to claim 14, wherein:
(a) the disease is an autoimmune or allergic disease;
(b) the disease is transplant rejection or graft-vs-host disease;
(c) the use is in suppressing an immune response;
(d) the disease is a neurodegenerative disease, optionally wherein the disease is amyotrophic lateral sclerosis (ALS);
(e) the disease is diabetes mellitus, optionally wherein the disease is type I diabetes mellitus;
(f) the disease is inflammation, an inflammatory skin disease or a neuroinflammatory disease;
(g) the disease is systemic lupus erythematosus (SLE).
